# EUROPEAN PATENT SPECIFICATION

(11) **EP 3 971 184 B1**
(45) Date of publication and mention of the grant of the patent: **03.04.2024**
(21) Application number: 20826825.0
(22) Date of filing: 18.06.2020
(51) Int. Cl.: C07D 209/08

(54) **METHOD FOR PREPARING INDOLE OR INDAZOLE COMPOUND**
VERFAHREN ZUR HERSTELLUNG VON INDOL- ODER INDAZOLVERBINDUNG
PROCÉDÉ DE PRÉPARATION D'UN COMPOSÉ D'INDOLE OU D'INDAZOLE

(30) Priority: 19.06.2019 KR 20190073019
(43) Date of publication of application: 23.03.2022
(73) Proprietor: LG Chem, Ltd., Seoul 07336 (KR)
(72) Inventor: LEE, Sang Dae, Daejeon 34122 (KR); PARK, Ae Ri, Daejeon 34122 (KR); KIM, Bong Chan, Daejeon 34122 (KR)
(74) Representative: Hoffmann Eitle
(86) International application number: PCT/KR2020/007894
(87) International publication number: WO 2020/256429

(56) References cited:
- EP-A1- 1 873 144
- KR-A- 20090 075 638
- KR-A- 20100 028 122
- KR-A- 20130 087 283
- KR-A- 20150 022 707
- US-A- 5 205 837

## Description

### [TECHNICAL FIELD]

This application claims priority to and the benefit of Korean Patent Application No. 10-2019-0073019, filed on June 19, 2019.

### Technical Field

The present invention relates to a method for preparing an indole or indazole compound containing an amine group, which is an intermediate structure necessary for the synthesis of a pharmaceutically useful indole or indazole compound.

### [BACKGROUND ART]

Many results of studies on compounds having an indole structure as a parent nucleus have been reported, and representative examples include, for example, PCT International Publication No. WO2006/112549, which reported that the compounds have the activity for the glucokinase, PCT International Publication No. WO1995/007276, which reported that the compounds are useful as anti-tumor agents and inhibitors against the production of cardiovascular system, and PCT International Publication No. WO2004/018428, which reported that the compounds can be used as antibiotics.

Among these, the present invention relates to a method for preparing an indole or indazole compound containing an amine group, as an intermediate structure necessary for obtaining an indole or indazole compound exhibiting an effect of prevention or treatment and amelioration for cellular necrosis and necrosis-associated diseases.

Conventionally, a method for preparing an indole or indazole compound containing an amine group using an iron (Fe) catalyst from an indole or indazole compound containing a nitro group has been utilized.

However, in this case, there have been limitations in that yield is lowered due to impurities generated in the reaction when there are various particle sizes of iron (Fe), stirring is not smooth during the reaction, or the reaction time is prolonged. In addition, there has been a limitation in that a reactor is coated with iron oxide produced after the reaction and the cost and time is required to clean the reactor.

Therefore, there is a need for developing a new cost-effective method capable of preparing an indole or indazole compound containing an amine group, which can solve the limitations of the conventional method using an iron (Fe) catalyst.

EP 1 873 144 A1 describes the synthesis of fused heterocyclic compounds used as glucokinase activators.

KR 2009-0075638 A describes compositions comprising indole and indazole compounds and the synthesis thereof.

KR 2010-0028122 A relates to chemical compounds used as anticoagulants and the synthesis thereof.

### [DISCLOSURE OF THE INVENTION]

### [TECHNICAL PROBLEM]

An aspect of the present invention provides a method for preparing an indole or indazole compound containing an amine group from an indole or indazole compound containing a nitro group, which can solve the limitations of the conventional method using an iron (Fe) catalyst and can cost-effectively prepare an indole or indazole compound containing an amine group.

### [TECHNICAL SOLUTION]

According to an aspect of the present invention, there is provided a method for preparing a compound represented by Formula 1 below, the method including a step for reducing a compound represented by Formula 2 below in the presence of iron(III) chloride hydrate, wherein the reducing step is performed by comprising an additional reducing agent, and wherein the reducing agent is at least one selected from the group consisting of NaBH₄, 9-borabicyclo[3.3.1]nonane (9-BBN), BH₃SMe₂, and LiBH₄: 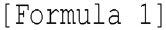 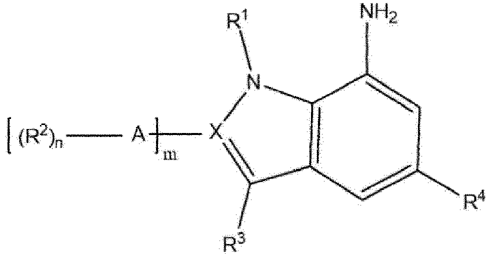
wherein, in Formula 1 above,
n is an integer of 1 to 3,
m is 0 or 1,
A represents C₃-C₈-cycloalkyl, phenyl, or 5-6 membered heteroaryl or heterocycle which each contains one or two heteroatoms selected from among N, O and S atoms,
X represents C or N, with the proviso that m is 0 when X is N and m is 1 when X is C,
R¹ represents hydrogen, C₁-C₆-alkyl or -(CH₂)ᵣNR⁹R¹⁰, wherein r is an integer of 2 to 5, R⁹ and R¹⁰ each independently represent hydrogen or C₁-C₃-alkyl, with the proviso that R¹ is hydrogen when X is N,
R² represents hydrogen, halogen or C₁-C₆-alkoxy, or represents -(CH₂)ₚC(O)₂R⁹, -(CH₂)ₚOR⁹, -(CH₂)ₚNR⁹R¹⁰, -NHR¹¹, - N(H)S(O)₂R⁹, or -NHC(O)₂R¹¹, or represents -(CH₂)ₚ-heterocycle-R¹¹ in which the heterocycle is a 5-6 membered ring containing one or two heteroatoms selected from among N, O, and S atoms, wherein p is an integer of 0 to 3, R⁹ and R¹⁰ are the same as defined above, and R¹¹ represents hydrogen, oxo, C₁-C₆-alkylcarbonyl, C₁-C₆-alkoxy or C₁-C₆-alkyl, or a 5-6 membered heterocycle containing one or two nitrogen atoms as a heteroatom,
R³ represents hydrogen, halogen, C₁-C₆-alkyl or phenyl, or -(CH₂)_{q}-heterocycle in which the heterocycle is a 5-6 membered ring containing one or two heteroatoms selected from among N and O atoms, wherein q is an integer of 1 to 3, with the proviso that R³ is hydrogen or phenyl when X is N,
R⁴ represents -Y¹R¹², wherein Y¹ is a direct bond or represents -(CR⁹R¹⁰)ₛY²-, wherein s is an integer of 0 to 3, and R¹⁰ are the same as defined above, and Y² is selected from the group consisting of -O-, -C(O)-, and -C(O)O-, and
R¹² is selected from the group consisting of hydrogen, halogen, C₁-C₆-alkyl, and -(CH₂)ₜB¹-R¹³, wherein t is an integer of 0 to 3, B¹ represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, with the proviso that R⁴ is hydrogen or C₁-C₆-alkyl when X is N,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

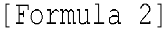

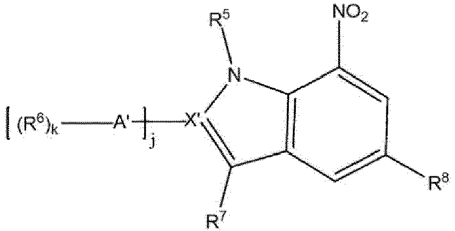

In Formula 2 above,
k is an integer of 1 to 3,
j is 0 or 1,
A' represents C₃-C₈-cycloalkyl, phenyl, or 5-6 membered heteroaryl or heterocycle which each contains one or two heteroatoms selected from among N, O and S atoms,
X' represents C or N, with the proviso that j is 0 when X' is N and j is 1 when X' is C,
R⁵ represents hydrogen, C₁-C₆-alkyl or -(CH₂)ₑNR¹⁴R¹⁵, wherein e is an integer of 2 to 5, R¹⁴ and R¹⁵ each independently represent hydrogen or C₁-C₃-alkyl, with the proviso that R⁵ is hydrogen when X' is N,
R⁶ represents hydrogen, halogen or C₁-C₆-alkoxy, or represents -(CH₂)_{f}C(O)₂R¹⁴, -(CH₂)_{f}OR¹⁴, -(CH₂)_{f}NR¹⁴R¹⁵, -NHR¹⁶, - N(H)S(O)₂R¹⁴, or -NHC(O)₂R¹⁶, or represents -(CH₂)_{f}-heterocycle-R¹⁶ in which the heterocycle is a 5-6 membered ring containing one or two heteroatoms selected from among N, O, and S atoms, wherein f is an integer of 0 to 3, R¹⁴ and R¹⁵ are the same as defined above, and R¹⁶ represents hydrogen, oxo, C₁-C₆-alkylcarbonyl, C₁-C₆-alkoxy or C₁-C₆-alkyl, or a 5-6 membered heterocycle containing one or two nitrogen atoms as a heteroatom,
R⁷ represents hydrogen, halogen, C₁-C₆-alkyl or phenyl, or -(CH₂)_{g}-heterocycle in which the heterocycle is a 5-6 membered ring containing one or two heteroatoms selected from among N and O atoms, wherein g is an integer of 1 to 3, with the proviso that R⁷ is hydrogen or phenyl when X' is N,
R⁸ represents -Y³R¹⁷, wherein Y³ is a direct bond or represents -(CR¹⁴R¹⁵)ₕY⁴-, wherein h is an integer of 0 to 3, R¹⁴ and R¹⁵ are the same as defined above, and Y⁴ is selected from the group consisting of -O-, -C(O)-, and -C(O)O-, and
R¹⁷ is selected from the group consisting of hydrogen, halogen, C₁-C₆-alkyl, and -(CH₂)ᵢB²-R¹⁸, wherein i is an integer of 0 to 3, B² represents a 5-6 membered heterocycle containing one or two heteroatoms selected from among N, O, and S atoms, or represents C₆-C₁₀-aryl, R¹⁸ represents hydrogen, cyano, halogen, hydroxy, oxo, thiol, carboxy or carboxy-C₁-C₆-alkyl, with the proviso that R⁸ is hydrogen or C₁-C₆-alkyl when X' is N,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

### [ADVANTAGEOUS EFFECTS]

The method for preparing an indole or indazole compound of the present invention can solve the limitations of the conventional iron (Fe) catalyst and can uniformly prepare a desired indole or indazole compound in excellent yield, and is cost-effective since iron(III) chloride hydrate much cheaper than iron (Fe) is used.

### [BEST MODE FOR CARRYING OUT THE INVENTION]

Hereinafter, the present invention will be described in more detail to aid in understanding the present invention.

Terms or words used in the specification and claims should not be interpreted as being limited to a conventional or dictionary meaning, and should be interpreted as the meaning and concept that accord with the technical spirit on the grounds of the principle that the inventor can appropriately define the concept of the term in order to explain the invention in the best way.

In the definition for the substituents of the compound of Formula (1) according to the present invention, the term "alkyl" refers to an aliphatic hydrocarbon radical. Alkyl may be "saturated alkyl" which does not include an alkenyl or alkynyl moiety, or "unsaturated alkyl" which includes at least one alkenyl or alkynyl moiety. The term "alkenyl" refers to a group containing at least one carbon-carbon double bond, and the term "alkynyl" refers to a group containing at least one carbon-carbon triple bond. Alkyl may be branched or linear when used alone or in a composite form such as alkoxy.

The alkyl group may have 1 to 20 carbon atoms unless otherwise defined. The alkyl group may be medium-sized alkyl having 1 to 10 carbon atoms. The alkyl group may be lower alkyl having 1 to 6 carbon atoms. A typical alkyl group includes, but is not limited to, methyl, ethyl, propyl, isopropyl, butyl, isobutyl, t-butyl, pentyl, hexyl, ethenyl, propenyl, butenyl, etc. For example, C₁-C₄-alkyl has 1 to 4 carbon atoms in the alkyl chain, and is selected from the group consisting of methyl, ethyl, propyl, iso-propyl, n-butyl, iso-butyl, sec-butyl and t-butyl.

The term "alkoxy" refers to alkyloxy having 1 to 10 carbon atoms unless otherwise defined.

The term "cycloalkyl" refers to a saturated aliphatic 3-10 membered ring unless otherwise defined. A typical cycloalkyl group includes, but is not limited to, cyclopropyl, cyclobutyl, cyclopentyl, cyclohexyl, etc.

The term "aryl" includes at least one ring having a covalent π electron system, for example, a monocyclic or fused polycyclic (i.e., rings that share adjacent pairs of carbon atoms) group. That is, unless otherwise defined herein, aryl refers to an aromatic 4-10 membered, preferably 6-10 membered, monocyclic or multicyclic ring including phenyl, naphthyl, etc.

The term "heteroaryl" refers to an aromatic 3-10 membered ring, preferably 4-8 membered ring, and more preferably 5-6 membered ring, which contains 1 to 3 heteroatoms selected from the group consisting of N, O and S and may be fused with benzo or C₃-C₈ cycloalkyl unless otherwise defined. Examples of monocyclic heteroaryl include, but are not limited to, thiazole, oxazole, thiophene, furan, pyrrole, imidazole, isoxazole, isothiazole, pyrazole, triazole, triazine, thiadiazole, tetrazole, oxadiazole, pyridine, pyridazine, pyrimidine, pyrazine and the like. Examples of bicyclic heteroaryl include, but are not limited to, indole, indoline, benzothiophene, benzofuran, benzimidazole, benzoxazole, benzisoxazole, benzthiazole, benzthiadiazole, benztriazole, quinoline, isoquinoline, purine, furopyridine and the like.

The term "heterocycle" refers to a 3-10 membered ring, preferably 4-8 membered ring, and more preferably 5-6 membered ring, which contains 1 to 3 heteroatoms selected from the group consisting of N, O and S and may be fused with benzo or C₃-C₈ cycloalkyl, and is saturated or contains one or two double bonds, unless otherwise defined. Examples of the heterocycle include, but are not limited to, pyrroline, pyrrolidine, imidazoline, imidazolidine, pyrazoline, pyrazolidine, pyran, piperidine, morpholine, thiomorpholine, piperazine, hydrofuran, and the like.

Unless otherwise defined, terms and abbreviations used herein may be interpreted as meanings commonly understood by those skilled in the art to which the present invention pertains.

The present invention relates to a method for preparing an indole or indazole compound containing an amine group which is an intermediate structure necessary for the synthesis of a pharmaceutically useful indole or indazole compound, the method being characterized by preparing a compound represented by Formula 1 by including a step for reducing a compound represented by Formula 2 in the presence of iron(III) chloride hydrate.

Iron(III) chloride hydrate herein can be referred to as Iron(III) chloride hexahydrate or ferric chloride hexahydrate, and is represented by FeCl₃·6H₂O.

The present invention can improve yield and reproducibility of a reaction by using iron(III) chloride hydrate instead of the conventional iron(Fe) catalyst and can solve the limitations caused by coating the reactor with products such as iron oxide.

The reducing step herein may be performed at a temperature of 0 °C to 65 °C. Specifically, the step for reducing may be performed at a temperature of 5 °C to 30 °C or 10 °C to 25 °C, and more specifically 20 °C to 25 °C.

The reducing step is performed by including an additional reducing agent. Specifically, the reducing agent is at least one selected from the group consisting of NaBH₄, 9-borabicyclo[3.3.1]nonane (9-BBN), BH₃SMe₂, and LiBH₄.

In addition, the preparation method of the present invention may further include a step for purifying the obtained compound after the step for reducing, wherein the purification may be performed by a solid purification method.

Specifically, the solid purification may include a step for filtering and washing the synthesized compound in any order, wherein water, a polar organic solvent, or a mixture thereof may be used for filtering and washing.

The polar organic solvent may specifically include at least one among alcohol and dichloromethane, and specifically, the alcohol may include at least one among methanol, ethanol, propanol, and butanol.

In addition, the filtering step is not specifically limited, and may be performed by a conventional manner. For example, the filtering step may be performed by using silica or celite filter.

Hereinafter, Formula 1, Formula 2, and related descriptions thereof will be described in detail.

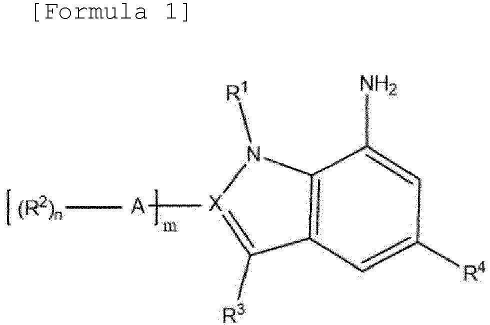

In Formula 1 above,
n is an integer of 1 to 3,
m is 0 or 1,
A represents C₃-C₈-cycloalkyl, phenyl, or 5-6 membered heteroaryl or heterocycle which each contains 1 or 2 heteroatoms selected from among N, O and S atoms,
X represents C or N, with the proviso that m is 0 when X is N and m is 1 when X is C,
R¹ represents hydrogen, C₁-C₆-alkyl or -(CH₂)ᵣNR⁹R¹⁰, wherein r is an integer of 2 to 5, and R¹⁰ each independently represent hydrogen or C₁-C₃-alkyl, with the proviso that R¹ is hydrogen when X is N,
R² represents hydrogen, halogen or C₁-C₆-alkoxy, or represents -(CH₂)ₚC(O)₂R⁹, -(CH₂)ₚOR⁹, -(CH₂)ₚNR⁹R¹⁰, -NHR¹¹, - N(H)S(O)₂R⁹, or -NHC(O)₂R¹¹, or represents -(CH₂)ₚ-heterocycle-R¹¹ in which the heterocycle is a 5-6 membered ring containing one or two heteroatoms selected from among N, O, and S atoms, wherein p is an integer of 0 to 3, and R¹⁰ are the same as defined above, and R¹¹ represents hydrogen, oxo, C₁-C₆-alkylcarbonyl, C₁-C₆-alkoxy or C₁-C₆-alkyl, or a 5-6 membered heterocycle containing one or two nitrogen atoms as a heteroatom,
R³ represents hydrogen, halogen, C₁-C₆-alkyl or phenyl, or -(CH₂)_{q}-heterocycle in which the heterocycle is a 5-6 membered ring containing one or two heteroatoms selected from among N and O atoms, wherein q is an integer of 1 to 3, with the proviso that R³ is hydrogen or phenyl when X is N,
R⁴ represents -Y¹R¹², wherein Y¹ is a direct bond or represents -(CR⁹R¹⁰)ₛY²-, wherein s is an integer of 0 to 3, and R¹⁰ are the same as defined above, and Y² is selected from the group consisting of -O-, -C(O)-, and -C(O)O-, and
R¹² is selected from the group consisting of hydrogen, halogen, C₁-C₆-alkyl, and -(CH₂)ₜB¹-R¹³, wherein t is an integer of 0 to 3, B¹ represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, with the proviso that R⁴ is hydrogen or C₁-C₆-alkyl when X is N,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

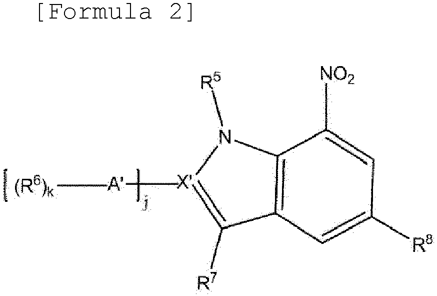

In Formula 2 above,
k is an integer of 1 to 3,
j is 0 or 1,
A' represents C₃-C₈-cycloalkyl, phenyl, or 5-6 membered heteroaryl or heterocycle which each contains 1 or 2 heteroatoms selected from among N, O and S atoms,
X' represents C or N, with the proviso that j is 0 when X' is N and j is 1 when X' is C,
R⁵ represents hydrogen, C₁-C₆-alkyl or -(CH₂)ₑNR¹⁴R¹⁵, wherein e is an integer of 2 to 5, R¹⁴ and R¹⁵ each independently represent hydrogen or C₁-C₃-alkyl, with the proviso that R⁵ is hydrogen when X' is N,
R⁶ represents hydrogen, halogen or C₁-C₆-alkoxy, or represents -(CH₂)_{f}C(O)₂R¹⁴, -(CH₂)_{f}OR¹⁴, -(CH₂)_{f}NR¹⁴R¹⁵, -NHR¹⁶, - N(H)S(O)₂R¹⁴, or -NHC(O)₂R¹⁶, or represents -(CH₂)_{f}-heterocycle-R¹⁶ in which the heterocycle is a 5-6 membered ring containing one or two heteroatoms selected from among N, O, and S atoms, wherein f is an integer of 0 to 3, R¹⁴ and R¹⁵ are the same as defined above, and R¹⁶ represents hydrogen, oxo, C₁-C₆-alkylcarbonyl, C₁-C₆-alkoxy or C₁-C₆-alkyl, or a 5-6 membered heterocycle containing one or two nitrogen atoms as a heteroatom,
R⁷ represents hydrogen, halogen, C₁-C₆-alkyl or phenyl, or -(CH₂)_{g}-heterocycle in which the heterocycle is a 5-6 membered ring containing one or two heteroatoms selected from among N and O atoms, wherein g is an integer of 1 to 3, with the proviso that R⁷ is hydrogen or phenyl when X' is N,
R⁸ represents -Y³R¹⁷, wherein Y³ is a direct bond or represents -(CR¹⁴R¹⁵)ₕY⁴-, wherein h is an integer of 0 to 3, R¹⁴ and R¹⁵ are the same as defined above, and Y⁴ is selected from the group consisting of -O-, -C(O)-, and -C(O)O-, and
R¹⁷ is selected from the group consisting of hydrogen, halogen, C₁-C₆-alkyl, and -(CH₂)ᵢB²-R¹⁸, wherein i is an integer of 0 to 3, B² represents a 5-6 membered heterocycle containing one or two heteroatoms selected from among N, O, and S atoms, or represents C₆-C₁₀-aryl, R¹⁸ represents hydrogen, cyano, halogen, hydroxy, oxo, thiol, carboxy or carboxy-C₁-C₆-alkyl, with the proviso that R⁸ is hydrogen or C₁-C₆-alkyl when X' is N,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

Specifically, in Formula 1 above, the R¹ may be hydrogen, C₁-C₆-alkyl, or di(C₁-C₃-alkyl)amino-C₂-C₃-alkyl.

In addition, the R² may represent hydrogen, halogen, carboxy, carboxy-C₁-C₃-alkyl, C₁-C₃-alkoxycarbonyl, C₁-C₃-alkoxycarbonyl-C₁-C₃-alkyl, hydroxy-C₁-C₃-alkyl optionally substituted by one oxo group, C₁-C₃-alkoxy, -(CH₂)ₚNR⁹R¹⁰, - NHR¹¹, -N(H)S(O)₂R⁹ or -NHC(O)₂R¹¹ or may be -(CH₂)ₚ-heterocycle-R¹¹, wherein heterocycle, p, R⁹, R¹⁰ and R¹¹ are the same as defined above.

Further, R³ may represent hydrogen, methyl or halogen, phenyl optionally substituted by C₁-C₃-alkoxy, or may be heterocyclyl-C₁-C₃-alkylene in which the heterocycle is a 5-6 membered ring containing one or two heteroatoms selected from N and O atoms and optionally substituted with one or two oxo groups.

In addition, the Y¹ may be selected from the group consisting of a direct bond, -O-, -C(O)-, and -CH₂C(O)-.

In addition, the R¹² may be selected from the group consisting of hydrogen, methyl, ethyl, phenyl, fluoro, chloro, 2-carboxy-pyrrolidin-1-yl, pyrrolidin-1-yl, 4-acetic acid-1,3-thiazolin-2-yl, -CH₂-(1,1-dioxo-thiomorpholin-4-yl) and - CH₂-(2-oxopiperazin-4-yl) .

In addition, in Formula 2 above, the R⁵ may be hydrogen, C₁-C₆-alkyl, or di(C₁-C₃-alkyl) amino-C₂-C₃-alkyl.

In addition, the R⁶ may represent hydrogen, halogen, carboxy, carboxy-C₁-C₃-alkyl, C₁-C₃-alkoxycarbonyl, C₁-C₃-alkoxycarbonyl-C₁-C₃-alkyl, hydroxy-C₁-C₃-alkyl optionally substituted by one oxo group, C₁-C₃-alkoxy, -(CH₂)_{f}NR¹⁴R¹⁵, - NHR¹⁶, -N(H)S(O)₂R¹⁴ or -NHC(O)₂R¹⁶ or may be -(CH₂)ₚ-heterocycle-R¹⁶, wherein heterocycle, f, R¹⁴, R¹⁵ and R¹⁶ are the same as defined above.

Further, R⁷ may represent hydrogen, methyl or halogen, phenyl optionally substituted by C₁-C₃-alkoxy, or may be heterocyclyl-C₁-C₃-alkylene in which the heterocycle is a 5-6 membered ring containing one or two heteroatoms selected from N and O atoms and optionally substituted with one or two oxo groups.

In addition, the Y³ may be selected from the group consisting of a direct bond, -O-, -C(O)-, and -CH₂C(O)-.

In addition, the R¹⁷ may be selected from the group consisting of hydrogen, methyl, ethyl, phenyl, fluoro, chloro, 2-carboxy-pyrrolidin-1-yl, pyrrolidin-1-yl, 4-acetic acid-1,3-thiazolin-2-yl, -CH₂-(1,1-dioxo-thiomorpholin-4-yl) and - CH₂-(2-oxopiperazin-4-yl) .

In addition, the compound represented by Formula 1 above may be represented by Formula (1a) or (1b) below: 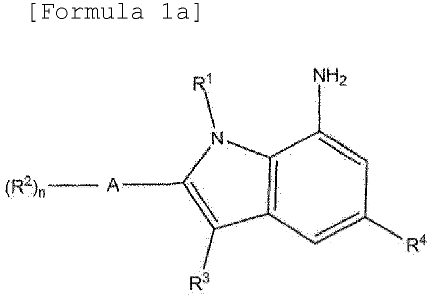 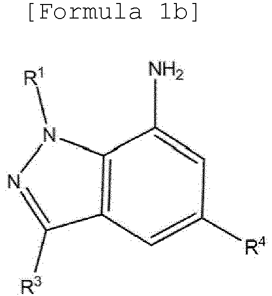 wherein, in Formulae above, n, A, R¹, R², R³ and R⁴ are the same as defined above.

In addition, the compound represented by Formula 2 above may be represented by Formula (2a) or (2b) below: 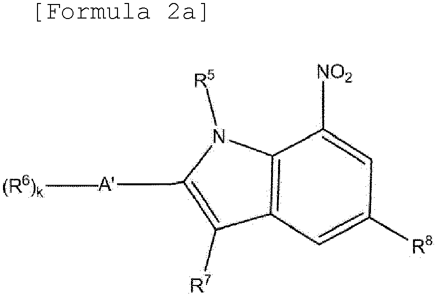 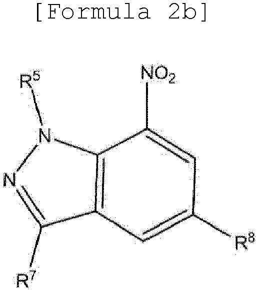 wherein, in Formulae above, k, A', R⁵, R⁶, R⁷, and R⁸ are the same as defined above.

For example, the compound represented by Formula 1 or Formula 1a above may be (4-((7-amino-2-phenyl-1H-indol-5-yl)methyl)thiomorpholine 1,1-dioxide, and the compound represented by Formula 2 or Formula 2a above may be (4-((7-nitro-2-phenyl-1H-indol-5-yl)methyl)thiomorpholine 1,1-dioxide.

In addition, the compound of Formula 2 above herein may be prepared by using the conventional known method.

For an indole compound in which X is C, a compound having a simple substituent (e.g., 7-nitro-indole, 2,3-dimethyl-7-nitro-indole, etc.) is commercially available, and most compounds may be synthesized by cyclizing an acetylene intermediate such as compound (8) of Reaction Scheme 1 below. In addition, compound (8) may be synthesized through a linking reaction from halobenzene compound (6) and acetylene compound (7).

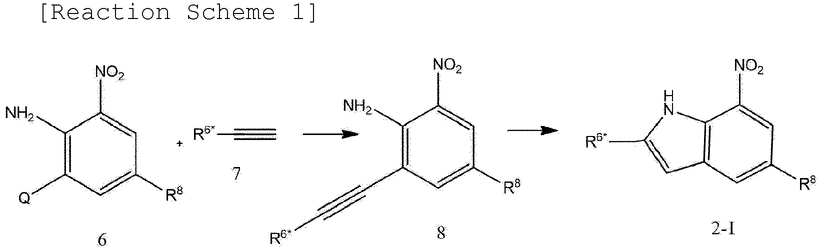

In Reaction Scheme 1 above,
R^{6*} represents (R⁶)k-A'-, k, A', R⁶, and R⁸ are the same as defined above, and Q represents iodine or bromine.

To obtain the compound of Formula 2 having a preferable R⁸ functional group, additional steps may be performed for substituting the R⁸ functional group with the preferable R⁸ functional group in compound (2-1) above, and this may use a conventional known method or a novel method, but the embodiment is not limited thereto.

Compound (2-1) above may be prepared by cyclizing acetylene compound (8) in the presence of a base such as KOBut, K₂CO₃, and DBU or a metal catalyst such as CuI and Pd(II).

Acetylene compound (8) may be prepared by adding a base in the presence of a metal catalyst, Pd(II), Cu(I), etc. are used as the metal catalyst, and Et₃N, Et₂N(iPr), DBU, N-methyl-morpholine, methyl-pyrrolidine, K₂CO₃, etc. are used as the base.

Acetylene compound (7) is typically commercially available, or may be prepared through a coupling reaction of acetylene in the presence of Pd(II) or Cu(I) and a base according to the known method described in Synthesis, 2004, 59-61; Bioorganic & Medicinal Chemistry, 13, 2005, 197-209; Journal of Organic Chemistry, 71, 2006, 167-175. The used acetylene is trimethylsilylacetylene or 2-methyl-3-butyn-2-ol, and the used base is diethylamine, triethylamine, diisopropylethyl amine, etc.

Halobenzene compound (6) may be synthesized from nitroaniline compound (13) by using iodine or bromine as shown in Reaction Scheme 2 below:

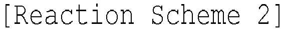

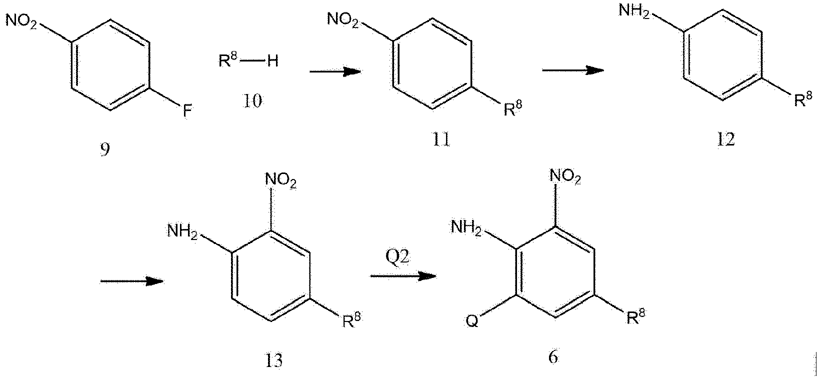

In Reaction Scheme 2 above, R⁸ is the same as defined above.

Nitroaniline compound (13) is commercially available, or may be synthesized through acetylation, nitration, and deacetylation reactions from nitro compound (11) or aniline compound (12), which is commercially available. Aniline compound (12), which is not commercially available, may be synthesized from nitro compound (11), and likewise, nitro compound (11) is also commercially available, or may be synthesized from 4-fluoro-nitrobenzene compound (9). In Reaction Scheme 2, compound (10) of Formula R⁸-H means alcohol and amine which are commercially available.

The halogenated material for preparing compound (6) may be selected from among iodine, bromine, iodine monobromide and iodine monochloride, and silver ions such as silver nitrate (AgNO₃), silver carbonate (AgCO₃), silver sulfate (Ag₂SO₄) may be used together.

Typical acetylation and nitration reactions may be used to obtain compound (13) from compound (12). The nitration reaction may be performed at a low temperature (-15 °C to 0 °C) using nitric acid as a stock solution, or the nitric acid may be used with various solvents, for example, dichloroethane or dichloromethane. In addition, the reduction reaction of the nitro group may be performed by using an acid catalyst and a metal, or a metal catalyst in the presence of hydrogen gas. Iron, zinc, lithium, sodium, and tin (typically, tin chloride) may be used as the metal, and an inorganic acid such as hydrochloric acid, sulfuric acid, nitric acid and phosphoric acid; an organic carboxylic acid such as acetic acid and trifluoroacetic acid; or ammonium chloride or the like may be used as the acid catalyst. In addition, a metal catalyst that may be used in a reduction reaction using a metal catalyst in the presence of hydrogen gas may include palladium, nickel, platinum, ruthenium, rhodium, etc., wherein the pressure of the hydrogen gas is typically 1 to 3 atmospheres.

Furthermore, the present invention relates to a method for preparing a compound represented by Formula 3 below, the method including the method for preparing the compound of Formula 1 followed by a step of reacting the compound represented by Formula 1 with a ketone or an aldehyde:

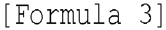

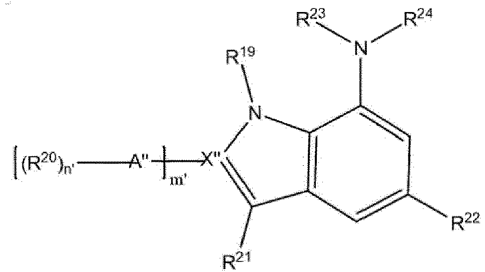

In Formula 3 above,
n' is an integer of 1 to 3,
m' is 0 or 1,
A" represents C₃-C₈-cycloalkyl, phenyl, or 5-6 membered heteroaryl or heterocycle which each contains one or two heteroatoms selected from among N, O and S atoms,
X" represents C or N, with the proviso that m' is 0 when X" is N and m' is 1 when X" is C,
R¹⁹ represents hydrogen, C₁-C₆-alkyl or -(CH₂)_{U}NR²⁵R²⁶, wherein u is an integer of 2 to 5, R²⁵ and R²⁶ each independently represent hydrogen or C₁-C₃-alkyl, with the proviso that R¹⁹ is hydrogen when X" is N,
R²⁰ represents hydrogen, halogen or C₁-C₆-alkoxy, or represents -(CH₂)ᵥC(O)₂R²⁵, -(CH₂)ᵥOR²⁵, -(CH₂)ᵥNR²⁵R²⁶, -NHR²⁷, - N(H)S(O)₂R²⁵, or -NHC(O)₂R²⁷, represents -(CH₂)ᵥ-heterocycle-R²⁷ in which the heterocycle is a 5-6 membered ring containing one or two heteroatoms selected from among N, O, and S atoms, wherein v is an integer of 0 to 3, R²⁵ and R²⁶ are the same as defined above, and R²⁷ represents hydrogen, oxo, C₁-C₆-alkylcarbonyl, C₁-C₆-alkoxy or C₁-C₆-alkyl, or a 5-6 membered heterocycle containing one or two nitrogen atoms as a heteroatom,
R²¹ represents hydrogen, halogen, C₁-C₆-alkyl or phenyl, or -(CH₂)_{w}-heterocycle in which the heterocycle is a 5-6 membered ring containing one or two heteroatoms selected from among N and O atoms, wherein w is an integer of 1 to 3, with the proviso that R²¹ is hydrogen or phenyl when X" is N,
R²² represents -Y⁵R²⁸, wherein Y⁵ is a direct bond or represents -(CR²⁵R²⁶)ₓY⁶-, wherein x is an integer of 0 to 3, R²⁵ and R²⁶ are the same as defined above, and Y⁶ is selected from the group consisting of -O-, -C(O)-, and -C(O)O-,
R²⁸ is selected from the group consisting of hydrogen, halogen, C₁-C₆-alkyl, and -(CH₂)_{y}B³-R²⁹, wherein y is an integer of 0 to 3, B³ represents a 5-6 membered heterocycle containing one or two heteroatoms selected from among N, O, and S atoms, or represents C₆-C₁₀-aryl, R²⁹ represents hydrogen, cyano, halogen, hydroxy, oxo, thiol, carboxy or carboxy-C₁-C₆-alkyl, with the proviso that R²² is hydrogen or C₁-C₆-alkyl when X" is N,
R²³ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms, with the proviso that R²³ is hydrogen when X" is N, and
R²⁴ represents -(CR²⁵R²⁶)ₒ-Z-D-W-R³⁰, wherein Z represents a direct bond or is selected from the group consisting of -C(O)- and -C(O)O-, D represents a direct bond, C₄-C₆-cycloalkyl, 5-6 membered heteroaryl containing one or two N atoms, or a 5-6 membered heterocycle containing one or two heteroatoms selected from among N, O and S atoms, W represents a direct bond, or -NR²⁵-, -C(O)-, -C(O)O-, - C(O)NR³¹- or -S(O)_{z}-, R³¹ represents hydrogen, C₁-C₃-alkyl or C₆-C₁₀-aryl, z is an integer of 1 or 2, o is an integer of 0 to 3, R²⁵ and R²⁶ are the same as defined above, R³⁰ represents hydrogen, hydroxy, C₁-C₆-alkyl, a 5-6 membered heterocycle containing one to three heteroatoms selected from among N, 0 and S atoms, or C₆-C₁₀-Ar-C₁-C₆-alkyl, with the proviso that when X" is N, R²⁴ represents C₄-C₆-cycloalkyl, or a 5-6 membered heterocycle containing one or two heteroatoms selected from among N, O, and S atoms,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

It should be interpreted that the compound of Formula 3 includes a pharmaceutically acceptable salt and isomer thereof. For the convenience of the description, these are simply expressed herein as the compound of Formula 3.

As used herein, "pharmaceutically acceptable salts" include acid addition salts formed by acids, which are capable of forming non-toxic acid addition salts containing pharmaceutically acceptable anions, for example, inorganic acids such as hydrochloric acid, sulfuric acid, nitric acid, phosphoric acid, hydrobromic acid and hydroiodic acid; organic carboxylic acids such as tartaric acid, formic acid, citric acid, acetic acid, trichloroacetic acid, trifluoroacetic acid, gluconic acid, benzoic acid, lactic acid, fumaric acid, maleic acid and salicylic acid; or sulfonic acids such as methanesulfonic acid, ethanesulfonic acid, benzenesulfonic acid, p-toluenesulfonic acid, and naphthalenesulfonic acid. In addition, pharmaceutically acceptable base addition salts, for example, alkali metal or alkaline earth metal salts formed by lithium, sodium, potassium, calcium, magnesium, etc.; amino acid salts with lysine, arginine, guanidine, etc.; or organic salts with dicyclohexylamine, N-methyl-D-glucamine, tris(hydroxymethyl)methylamine, diethanolamine, choline, triethylamine, etc. are included. The compounds of Formula 3 according to the present invention may be converted to salts thereof by means of any conventional methods, and the salt formation may be easily carried out by a person skilled in the art based on the structure of Formula 3 above without additional explanations thereon.

In addition, the term 'isomer' herein means those having the same chemical or molecular formula as, but optically or sterically different from, the compounds of Formula 3, or salts thereof. The compounds of formula 3 according to the present invention may have an asymmetric carbon center, so that the compounds may exist in the form of optical isomer (R or S isomer), racemate, mixture of diastereomers, or individual diastereomer, etc. When the compounds have a double bond, the compounds may also exist in the form of geometric isomer (trans or cis isomer). All these isomers and mixtures thereof are also included in the scope of the present invention.

### [MODE FOR CARRYING OUT THE INVENTION]

Hereinafter, the present invention will be described in more detail according to the examples. However, the following examples are intended to illustrate the present invention, and the scope of the present invention is not limited thereto.

### Example

### Preparation Example 1

### Synthesis of 4-((7-nitro-2-phenyl-1H-indol-5-yl)methyl)thiomorpholine 1,1-dioxide

### 1) Synthesis of (4-Amino-3-iodo-5-nitrobenzoic Acid)

Commercially available (4-amino-3-nitrobenzoic acid) (manufactured by Sinochem Ningbo Co., Ltd.) (5.0 kg), NIS (N-Iodosuccinimide, 9.3 kg), H₂SO₄ (0.50 kg) and THF (25.0 L) were added to a reactor at room temperature and stirred, and then the reactor was heated to a temperature of 80 °C and stirred for 2 hours. Then, the reaction was terminated when the reaction solution was analyzed by HPLC to show 5.0% or less of the peak of (4-amino-3-nitrobenzoic acid).

After confirming the completion of the reaction, the temperature of the reactor was cooled to room temperature, and then dichloromethane (DCM, 50.0 L) was added to a concentrated solution obtained by distillation under reduced pressure to form a solid. The formed solid was stirred at room temperature for 1 hour, filtered and washed [1st: DCM (40.0 L), 2nd: H₂O (30.0 L), 3rd: EtOH/H₂O = 3/7, 30.0 L], and then dried under N₂ pressure for 16 hours to synthesize (4-amino-3-iodo-5-nitrobenzoic acid) (7.6 kg, Yield: 89.8%, Purity: 96.4%).

¹H NMR (500MHz, DMSO-d₆) δ 13.1 (br s, 1H), 8.52 (d, J=1.8 Hz, 1H) 8.36 (d, J=1.8 Hz, 1H), 7.50 (s, 2H).

### 2) Synthesis of (7-Nitro-2-phenyl-1H-indole-5-carboxylic Acid)

(4-amino-3-iodo-5-nitrobenzoic acid) (7.6 kg), triethylamine (TEA, 7.5 kg), phenylacetylene (3.0 kg), and 1,4-dioxane (76.0 L) were added to a reactor at room temperature and stirred, and then CuI [Copper(I) Iodide, 47.0 g] and Pd(PPh₃)₂Cl₂ (173.0 g), which are reaction catalysts, were added thereto, and the reactor was heated to a temperature of 60 °C. The reaction mixture was stirred for 2 hours. Then, the reaction was terminated when the reaction solution was analyzed by HPLC to show 1.0% or less of the peak of (4-amino-3-iodo-5-nitrobenzoic acid).

Then, 1,8-diazabicyclo[5.4.0]undec-7-ene (DBU, 22.5 kg) was added to the reaction mixture, the reactor was heated to a temperature of 110 °C, the reaction mixture was stirred for 18 hours, and the reaction was then terminated. After the reaction mixture was cooled to room temperature, pH was adjusted to 3 with a 3N-HCl aqueous solution (114.0 L), H₂O (38.0 L) was added thereto, the mixture was stirred for 2 hours, filtered and washed [1st: H₂O (38.0 L), 2nd: EtOH/H₂O = 3/7, 23.0 L], and then dried under N₂ pressure for 16 hours to synthesize (7-nitro-2-phenyl-1H-indole-5-carboxylic acid) (6.1 Kg, Yield: 87.4%, Purity: 98.0%).

¹H NMR (500MHz, DMSO-d₆) δ 11.6 (s, 1H), 8.03 (s, 1H), 7.96 (m, 3H), 7.46 (t, J= 7.7 Hz, 2H) 7.36 (d, J= 7.3 Hz, 1H), 7.10 (s, 1H)

### 3) Synthesis of (7-Nitro-2-phenyl-1H-indol-5-yl)methanol)

(7-nitro-2-phenyl-1H-indole-5-carboxylic acid) (5.8 kg) and THF (61 L) were added to a reactor at room temperature and stirred, and then the reaction mixture was cooled to 12 ± 2 °C, and 5M BH₃-DMS (12.3 L) was slowly added dropwise thereto so that the internal temperature thereof did not exceed 40 °C. The dropwise addition was completed, the reactor was heated to a temperature of 40 °C, and then the reaction mixture was stirred for 2 hours. Then, the reaction was terminated when the reaction solution was analyzed by HPLC to show 1.0% or less of the peak of (7-nitro-2-phenyl-1H-indole-5-carboxylic acid).

After confirming the completion of the reaction, H₂O (55 L) was slowly added dropwise to the reaction mixture, and then the mixture was separated into layers to store an organic layer, and the water layer was extracted with EtOAc (29 L), mixed with the stored organic layer, and distilled under reduced pressure. EtOH/H₂O (6.1 L/12.2 L) was added to the obtained concentrate, stirred for 2 hours, and then filtered. The resultant was washed with a washing solution (EtOH/H₂O = 1/3, 9.0 L) and then dried under N₂ pressure for 16 hours to synthesize (7-nitro-2-phenyl-1H-indol-5-yl)methanol (4.6 kg, yield: 83.5%, purity: 98.4%).

¹H NMR (500MHz, DMSO-d₆) δ 11.6 (s, 1H), 8.07 (s, 1H), 8.00 (m, 3H), 7.49 (t, J= 7.7 Hz, 2H) 7.40 (d, J= 7.3 Hz, 1H), 7.14 (s, 1H), 5.42 (t, J= 6.7 Hz, 1H), 4.66 (d, J= 6.7 Hz, 2H) .

### 4) Synthesis of 4-((7-nitro-2-phenyl-1H-indol-5-yl)methyl)thiomorpholine 1,1-dioxide

7-nitro-2-phenyl-1H-indol-5-yl)methanol (4.6 kg) and THF (46 L) were added to a reactor and stirred at room temperature, the reactor was cooled to a temperature of 0 °C, and then PBr₃ (2.8 kg) was added while the internal temperature of the reaction was maintained below 20 °C. After the completion of the addition, the temperature of the reactor was raised to 25 °C, the mixture was stirred for 1 hour, and the reaction was terminated when the reaction mixture was analyzed by sampling to show 1.0% or less of the peak of 5-(hydroxymethyl)-7-nitro-2-phenyl-1H-indole.

The completion of the reaction was confirmed, the reaction mixture was cooled to 0 °C, and 1,1-dioxothiomorpholine HCl (4.4 kg) and N,N-diisopropylethylamine (DIPEA) (8.0 kg) were added while the internal temperature of the reaction was maintained below 20 °C. After the completion of the addition, the temperature of the reactor was raised to 50 °C, the mixture was stirred for 4 hours, and the reaction was terminated when the reaction mixture was analyzed by sampling to show 1.0% or less of the peak of 5-(bromomethyl)-7-nitro-2-phenyl-1H-indole.

After confirming the completion of the reaction, the reactor was cooled to a temperature of 0 °C, water (46.0 L) was added thereto, the reaction mixture was stirred, and the organic solvent was distilled under reduced pressure. After the distillation under reduced pressure, ethyl acetate (EtOAc) (48.0 L) was additionally added thereto, and the organic solvent was distilled under reduced pressure again to obtain 4-((7-nitro-2-phenyl-1H-indol-5-yl)methyl)thiomorpholine 1,1-dioxide as a solid, and then filtered. The resultant was washed with water (26.0 L) and ethanol (26.0 L) and dried under N₂ pressure for 16 hours to synthesize crude 4-((7-nitro-2-phenyl-1H-indol-5-yl)methyl)thiomorpholine 1,1-dioxide (6.4 kg, purity: 92.9%).

For purification, crude 4-((7-nitro-2-phenyl-1H-indol-5-yl)methyl)thiomorpholine 1,1-dioxide above was added to a mixture of DMF (6.0 L) and toluene (60.0 L), stirred, heated to 120 °C, stirred for 2 hours, and then stirred for another 2 hours at room temperature again. Toluene (60.0 L) was additionally added thereto and stirred for 2 another hours, and then a solid in the solution was filtered and washed with toluene (20.0 L) to synthesize 4-((7-nitro-2-phenyl-1H-indol-5-yl)methyl)thiomorpholine 1,1-dioxide (5.1 kg, yield: 77.0% overall, purity: 98.4%).

¹H NMR (500MHz, DMSO-d₆) δ 11.63 (s, 1H), 8.07 (s, 1H), 8.04 (m, 3H), 7.49 (t, J= 7.7 Hz, 2H) 7.38 (d, J= 7.3 Hz, 1H), 7.14 (s, 1H), 3.79 (s, 2H), 3.12 (m, 4H), 2.84 (m, 4H).

### Example 1

### Synthesis of 4-((7-amino-2-phenyl-1H-indol-5-yl)methyl)thiomorpholine 1,1-dioxide

(4-((7-amino-2-phenyl-1H-indol-5-yl)methyl)thiomorpholine 1,1-dioxide) (0.345 kg) of Preparation Example 1 above, THF/MeOH (2:1, 5.18 L) and FeCl₃·6H₂O (2.4 g) were added to a reactor at room temperature, stirred for 30 minutes, and then the reactor was cooled to a temperature of 0 °C to 5 °C. NaBH₄ (0.1 kg) was slowly added thereto for 30 minutes, the reactor was heated to a temperature of 20 °C to 25 °C, stirred for 2 hours, and the reaction was terminated when the reaction mixture was analyzed to show 0.5% PAR or less of the peak of (4-((-7-nitro-2-phenyl-1H-indol-5-yl)methyl)thiomorpholine 1,1-dioxide).

The completion of the reaction was confirmed, THF (3.0 L) and H₂O (1.73 L) were added to the reaction mixture and stirred for 30 minutes, and then filtered with celite, and the resultant was washed with THF/H₂O (1:1, 1.0 L). The filtrate was under reduced pressure and THF was distilled to form a solid, and the reaction mixture was stirred at room temperature for 2 hours. The resultant was filtered and washed with the washing solution [1st H₂O: 1.73 L, 2nd ethanol/H₂O = 1/3, 1.73 L], and then dried under N₂ pressure for 16 hours to obtain the title compound [0.31 kg (yield: 97.53%, purity: 97.64% PAR)].

¹H NMR (500MHz, DMSO-d₆) δ 10.89 (s, 1H), 7.80 (m, 2H), 7.48 (t, J= 7.7 Hz, 2H) 7.30 (d, J= 7.3 Hz, 1H), 6.74 (m, 2H), 6.34 (s, 1H), 5.15 (s, 2H), 3.07 (m, 4H), 2.87 (m, 4H).

### Comparative Example 1

### Synthesis of 4-((7-amino-2-phenyl-1H-indol-5-yl)methyl)thiomorpholine 1,1-dioxide

4-((7-amino-2-phenyl-1H-indol-5-yl)methyl)thiomorpholine 1,1-dioxide (0.60 kg) of Preparation Example 1 above, THF/MeOH/H₂O (1:1:1, 3.0 L), and NH₄Cl (124 g) were added to a reactor at room temperature and stirred, and then Fe (480 g) was added thereto. The reactor was heated to a temperature of 60 °C, the reaction mixture was stirred for 1 hour, and then the reaction was terminated when the reaction mixture was analyzed by sampling to show 0.5% PAR or less of the peak of 4-((7-nitro-2-phenyl-1H-indol-5-yl)methyl)thiomorpholine 1,1-dioxide.

The completion of the reaction was confirmed, THF (3.0 L) was added to the reaction mixture and stirred for 10 minutes, and then filtered with celite, and the resultant was washed with THF/H₂O (1:1, 3.0 L). The filtrate was under reduced pressure and THF and MeOH were distilled to form a solid, and then the reaction mixture was stirred at room temperature for 2 hours. The resultant was filtered and washed with the washing solution [1st H₂O: 500 mL, 2nd EtOH/H₂O = 1/3, 1.73 L], and then dried under N₂ pressure for 16 hours to obtain the title compound [0.45 kg (yield: 81.3%, purity: 97.2% PAR)].

From the above results, it is confirmed that Example 1 of the present invention may obtain a high-purity compound in excellent yield compared to Comparative Example 1. Specifically, the indole compound of Example 1 has a yield of 97.53% and a purity of 97.64%, while the indole compound of Comparative Example 1 has a yield of 81.3% and a purity of 97.2%.

Moreover, according to an embodiment of the present invention, as described above, the reactor is not coated because there are no iron oxides produced after the reaction, and since iron(III) chloride (FeCl₃) hydrate used as a catalyst in Example is much cheaper than iron (Fe) in Comparative Example, the method according to the present invention is cost-effective.

## Claims

1. A method for preparing a compound represented by Formula 1 below, the method comprising a step for reducing a compound represented by Formula 2 below in the presence of iron(III) chloride hydrate,
wherein the reducing step is performed by comprising an additional reducing agent, and
wherein the reducing agent is at least one selected from the group consisting of NaBH₄, 9-borabicyclo[3.3.1]nonane (9-BBN), BH₃SMe₂, and LiBH₄: 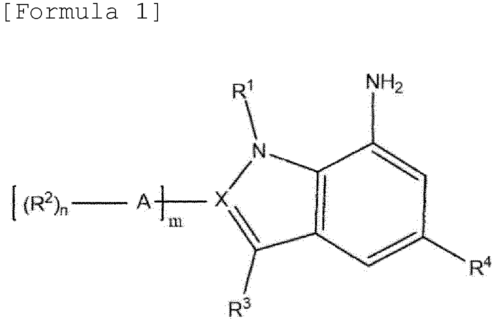
wherein, in Formula 1 above,
n is an integer of 1 to 3,
m is 0 or 1,
A represents C₃-C₈-cycloalkyl, phenyl, or 5-6 membered heteroaryl or heterocycle which each contains one or two heteroatoms selected from among N, O and S atoms,
X represents C or N, with the proviso that m is 0 when X is N and m is 1 when X is C,
R¹ represents hydrogen, C₁-C₆-alkyl or -(CH₂)ᵣNR⁹R¹⁰, wherein r is an integer of 2 to 5, and R¹⁰ each independently represent hydrogen or C₁-C₃-alkyl, with the proviso that R¹ is hydrogen when X is N,
R² represents hydrogen, halogen or C₁-C₆-alkoxy, or represents -(CH₂)ₚC(O)₂R⁹, -(CH₂)ₚOR⁹, -(CH₂)ₚNR⁹R¹⁰, -NHR¹¹, - N(H)S(O)₂R⁹, or -NHC(O)₂R¹¹, or represents -(CH₂)ₚ-heterocycle-R¹¹ in which the heterocycle is a 5-6 membered ring containing one or two heteroatoms selected from among N, O, and S atoms, wherein p is an integer of 0 to 3, and R¹⁰ are the same as defined above, and R¹¹ represents hydrogen, oxo, C₁-C₆-alkylcarbonyl, C₁-C₆-alkoxy or C₁-C₆-alkyl, or a 5-6 membered heterocycle containing one or two nitrogen atoms as a heteroatom,
R³ represents hydrogen, halogen, C₁-C₆-alkyl or phenyl, or -(CH₂)_{q}-heterocycle in which the heterocycle is a 5-6 membered ring containing one or two heteroatoms selected from among N and O atoms, wherein q is an integer of 1 to 3, with the proviso that R³ is hydrogen or phenyl when X is N,
R⁴ represents -Y¹R¹², wherein Y¹ is a direct bond or represents -(CR⁹R¹⁰)ₛY²-, wherein s is an integer of 0 to 3, and R¹⁰ are the same as defined above, and Y² is selected from the group consisting of -O-, -C(O)-, and -C(O)O-, and
R¹² is selected from the group consisting of hydrogen, halogen, C₁-C₆-alkyl, and - (CH₂)ₜB¹-R¹³, wherein t is an integer of 0 to 3, B¹ represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, with the proviso that R⁴ is hydrogen or C₁-C₆-alkyl when X is N,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo, and 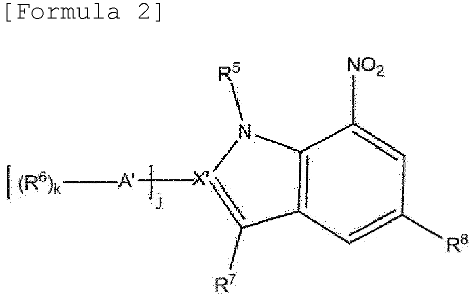
wherein, in Formula 2 above,
k is an integer of 1 to 3,
j is 0 or 1,
A' represents C₃-C₈-cycloalkyl, phenyl, or 5-6 membered heteroaryl or heterocycle which each contains 1 or 2 heteroatoms selected from among N, O and S atoms,
X' represents C or N, with the proviso that j is 0 when X' is N and j is 1 when X' is C,
R⁵ represents hydrogen, C₁-C₆-alkyl or -(CH₂)ₑNR¹⁴R¹⁵, wherein e is an integer of 2 to 5, R¹⁴ and R¹⁵ each independently represent hydrogen or C₁-C₃-alkyl, with the proviso that R⁵ is hydrogen when X' is N,
R⁶ represents hydrogen, halogen or C₁-C₆-alkoxy, or represents -(CH₂)_{f}C(O)₂R¹⁴, -(CH₂)_{f}OR¹⁴, -(CH₂)_{f}NR¹⁴R¹⁵, -NHR¹⁶, - N(H)S(O)₂R¹⁴, or -NHC(O)₂R¹⁶, or represents -(CH₂)_{f}-heterocycle-R¹⁶ in which the heterocycle is a 5-6 membered ring containing one or two heteroatoms selected from among N, O, and S atoms, wherein f is an integer of 0 to 3, R¹⁴ and R¹⁵ are the same as defined above, and R¹⁶ represents hydrogen, oxo, C₁-C₆-alkylcarbonyl, C₁-C₆-alkoxy or C₁-C₆-alkyl, or a 5-6 membered heterocycle containing one or two nitrogen atoms as a heteroatom,
R⁷ represents hydrogen, halogen, C₁-C₆-alkyl or phenyl, or -(CH₂)_{g}-heterocycle in which the heterocycle is a 5-6 membered ring containing one or two heteroatoms selected from among N and O atoms, wherein g is an integer of 1 to 3, with the proviso that R⁷ is hydrogen or phenyl when X' is N,
R⁸ represents -Y³R¹⁷, wherein Y³ is a direct bond or represents -(CR¹⁴R¹⁵)ₕY⁴-, wherein h is an integer of 0 to 3, R¹⁴ and R¹⁵ are the same as defined above, and Y⁴ is selected from the group consisting of -O-, -C(O)-, and -C(O)O-, and
R¹⁷ is selected from the group consisting of hydrogen, halogen, C₁-C₆-alkyl, and - (CH₂)ᵢB²-R¹⁸, wherein i is an integer of 0 to 3, B² represents a 5-6 membered heterocycle containing one or two heteroatoms selected from among N, O, and S atoms, or represents C₆-C₁₀-aryl, R¹⁸ represents hydrogen, cyano, halogen, hydroxy, oxo, thiol, carboxy or carboxy-C₁-C₆-alkyl, with the proviso that R⁸ is hydrogen or C₁-C₆-alkyl when X' is N,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

2. The method of claim 1, wherein the iron(III) chloride hydrate is ferric chloride hexahydrate (FeCl₃·6H₂O).

3. The method of claim 1, wherein the reducing step is performed at a temperature of 0 °C to 65 °C.

4. The method of claim 1, wherein the compound represented by Formula 1 above is represented by Formula (1a) or (1b) below: 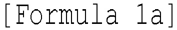 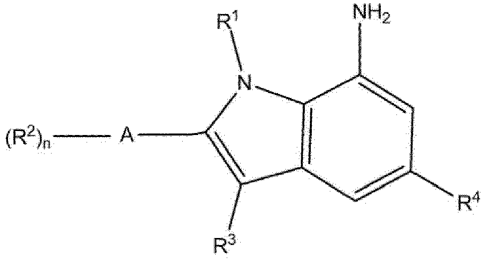 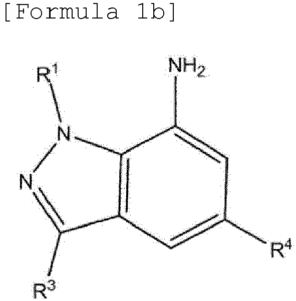 wherein, in Formulae above, n, A, R¹, R², R³ and R⁴ are the same as defined in claim 1.

5. The method of claim 1, wherein R¹ represents hydrogen, C₁-C₆-alkyl, or di(C₁-C₃-alkyl)amino-C₂-C₃-alkyl.

6. The method of claim 1, wherein R² represents hydrogen, halogen, carboxy, carboxy-C₁-C₃-alkyl, C₁-C₃-alkoxycarbonyl, C₁-C₃-alkoxycarbonyl-C₁-C₃-alkyl, hydroxy-C₁-C₃-alkyl optionally substituted by one oxo group, C₁-C₃-alkoxy, - (CH₂)ₚNR⁹R¹⁰, -NHR¹¹, -N(H)S(O)₂R⁹, or -NHC(O)₂R¹¹ or may be - (CH₂)ₚ-heterocycle-R¹¹, wherein heterocycle, p, R⁹, R¹⁰ and R¹¹ are the same as defined in claim 1.

7. The method of claim 1, wherein R³ represents hydrogen, methyl or halogen, phenyl optionally substituted by C₁-C₃-alkoxy, or heterocyclyl-C₁-C₃-alkylene in which the heterocycle is a 5-6 membered ring containing one or two heteroatoms selected from N and O atoms and optionally substituted with one or two oxo groups.

8. The method of claim 1, wherein Y¹ is selected from the group consisting of a direct bond, -O-, -C(O)-, and -CH₂C(O)-.

9. The method of claim 1, wherein R¹² is selected from the group consisting of hydrogen, methyl, ethyl, phenyl, fluoro, chloro, 2-carboxy-pyrrolidin-1-yl, pyrrolidin-1-yl, 4-acetic acid-1,3-thiazolin-2-yl, -CH₂-(1,1-dioxo-thiomorpholin-4-yl) and -CH₂-(2-oxopiperazin-4-yl).

10. The method of claim 1, wherein the compound represented by Formula 1 is (4-((7-amino-2-phenyl-1H-indol-5-yl)methyl)thiomorpholine 1,1-dioxide, and the compound represented by Formula 2 is (4-((7-nitro-2-phenyl-1H-indol-5-yl)methyl)thiomorpholine 1,1-dioxide.

11. A method for preparing a compound represented by Formula 3 below, the method comprising the method of claim 1 followed by a step of reacting the compound represented by Formula 1 with a ketone or an aldehyde: 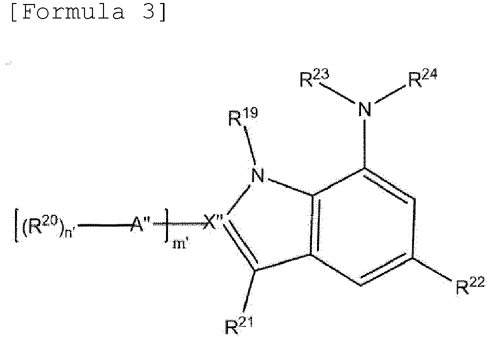
wherein, in Formula 3 above,
n' is an integer of 1 to 3,
m' is 0 or 1,
A" represents C₃-C₈-cycloalkyl, phenyl, or 5-6 membered heteroaryl or heterocycle which each contains one or two heteroatoms selected from among N, O and S atoms,
X" represents C or N, with the proviso that m' is 0 when X" is N and m' is 1 when X" is C,
R¹⁹ represents hydrogen, C₁-C₆-alkyl or -(CH₂)_{U}NR²⁵R²⁶, wherein u is an integer of 2 to 5, R²⁵ and R²⁶ each independently represent hydrogen or C₁-C₃-alkyl, with the proviso that R¹⁹ is hydrogen when X" is N,
R²⁰ represents hydrogen, halogen or C₁-C₆-alkoxy, or represents -(CH₂)ᵥC(O)₂R²⁵, -(CH₂)ᵥOR²⁵, -(CH₂)ᵥNR²⁵R²⁶, -NHR²⁷, - N(H)S(O)₂R²⁵, or -NHC(O)₂R²⁷, or represents -(CH₂)ᵥ-heterocycle-R²⁷ in which the heterocycle is a 5-6 membered ring containing one or two heteroatoms selected from among N, O, and S atoms, wherein v is an integer of 0 to 3, R²⁵ and R²⁶ are the same as defined above, and R²⁷ represents hydrogen, oxo, C₁-C₆-alkylcarbonyl, C₁-C₆-alkoxy or C₁-C₆-alkyl, or a 5-6 membered heterocycle containing one or two nitrogen atoms as a heteroatom,
R²¹ represents hydrogen, halogen, C₁-C₆-alkyl or phenyl, or -(CH₂)_{w}-heterocycle in which the heterocycle is a 5-6 membered ring containing one or two heteroatoms selected from among N and O atoms, wherein w is an integer of 1 to 3, with the proviso that R²¹ is hydrogen or phenyl when X" is N,
R²² represents -Y⁵R²⁸, wherein Y⁵ is a direct bond or represents -(CR²⁵R²⁶)ₓY⁶-, wherein x is an integer of 0 to 3, R²⁵ and R²⁶ are the same as defined above, and Y⁶ is selected from the group consisting of -O-, -C(O)-, and -C(O)O-,
R²⁸ is selected from the group consisting of hydrogen, halogen, C₁-C₆-alkyl, and -(CH₂)_{y}B³-R²⁹, wherein y is an integer of 0 to 3, B³ represents a 5-6 membered heterocycle containing one or two heteroatoms selected from among N, O, and S atoms, or represents C₆-C₁₀-aryl, R²⁹ represents hydrogen, cyano, halogen, hydroxy, oxo, thiol, carboxy or carboxy-C₁-C₆-alkyl, with the proviso that R²² is hydrogen or C₁-C₆-alkyl when X" is N,
R²³ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms, with the proviso that R²³ is hydrogen when X" is N, and
R²⁴ represents -(CR²⁵R²⁶)ₒ-Z-D-W-R³⁰, wherein Z represents a direct bond or is selected from the group consisting of - C(O)- and -C(0)0-, D represents a direct bond, C₄-C₆-cycloalkyl, 5-6 membered heteroaryl containing one or two N atoms, or a 5-6 membered heterocycle containing one or two heteroatoms selected from among N, O and S atoms, W represents a direct bond, or -NR²⁵-, -C(O)-, -C(O)O-, - C(O)NR³¹- or -S(O)_{z}-, R³¹ represents hydrogen, C₁-C₃-alkyl or C₆-C₁₀-aryl, z is an integer of 1 or 2, o is an integer of 0 to 3, R²⁵ and R²⁶ are the same as defined above, R³⁰ represents hydrogen, hydroxy, C₁-C₆-alkyl, a 5-6 membered heterocycle containing one to three heteroatoms selected from among N, 0 and S atoms, or C₆-C₁₀-Ar-C₁-C₆-alkyl, with the proviso that when X" is N, R²⁴ represents C₄-C₆-cycloalkyl, or a 5-6 membered heterocycle containing one or two heteroatoms selected from among N, O, and S atoms,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

## Patentansprüche

1. Verfahren zur Herstellung einer durch die nachstehende Formel 1 dargestellten Verbindung, wobei die Verfahren einen Schritt zum Reduzieren einer durch die nachstehende Formel 2 dargestellten Verbindung in Gegenwart von Eisen(III)-chlorid-Hydrat umfasst,
wobei der Reduktionsschritt durchgeführt wird, indem er ein zusätzliches Reduktionsmittel umfasst, und
wobei das Reduktionsmittel mindestens eines, ausgewählt aus der Gruppe, bestehend aus NaBH₄, 9-Borabicyclo[3.3.1]nonan (9-BBN), BH₃SMe₂ und LiBH₄, ist: 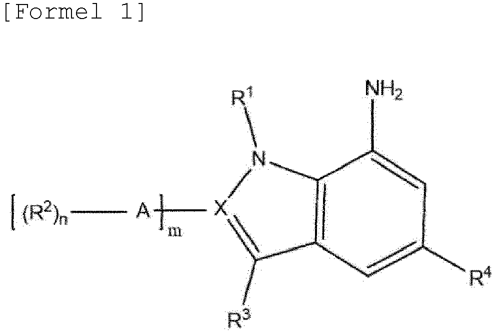
wobei in der obigen Formel 1
n eine ganze Zahl von 1 bis 3 ist,
m 0 der 1 ist,
A C₃-C₈-Cycloalkyl, Phenyl oder ein 5-6-gliedriges/gliedriger Heteroaryl oder Heterozyklus darstellt, das/der jeweils ein oder zwei Heteroatome, ausgewählt unter N-, O- und S-Atomen, enthält,
X C oder N darstellt, mit der Maßgabe, dass m 0 ist, wenn X N ist, und m 1 ist, wenn X C ist,
R¹ Wasserstoff, C₁-C₆-Alkyl oder -(CH₂)ᵣNR⁹R¹⁰ darstellt, worin r eine ganze Zahl von 2 bis 5 ist, und R¹⁰ jeweils unabhängig Wasserstoff oder C₁-C₃-Alkyl darstellen, mit der Maßgabe, dass R¹ Wasserstoff ist, wenn X N ist,
R² Wasserstoff, Halogen oder C₁-C₆-Alkoxy darstellt oder -(CH₂)ₚC(O)₂R⁹, -(CH₂)ₚOR⁹, -(CH₂)ₚNR⁹R¹⁰, -NHR¹¹, -N(H)S(O)₂R⁹ oder -NHC(O)₂R¹¹ darstellt oder -(CH₂)ₚ-Heterozyklus-R¹¹ darstellt, worin der Heterozyklus ein 5-6-gliedriger Ring ist, der ein oder zwei Heteroatome, ausgewählt unter N-, O- und S-Atomen, enthält, wobei p eine ganze Zahl von 0 bis 3 ist, R⁹ und R¹⁰ dieselben wie oben definiert sind und R¹¹ Wasserstoff, Oxo, C₁-C₆-Alkylcarbonyl, C₁-C₆-Alkoxy oder C₁-C₆-Alkyl oder einen 5-6-gliedriger Heterozyklus, der ein oder zwei Stickstoffatome als Heteroatom enthält, darstellt,
R⁹ Wasserstoff, Halogen, C₁-C₆-Alkyl oder Phenyl oder -(CH₂)_{q}-Heterozyklus darstellt, worin der Heterozyklus ein 5-6-gliedriger Ring ist, der ein oder zwei Heteroatome, ausgewählt aus N- und O-Atomen, enthält, wobei q eine ganze Zahl von 1 bis 3 ist, mit der Maßgabe, dass R³ Wasserstoff oder Phenyl ist, wenn X N ist,
R⁴ -Y¹R¹² darstellt, wobei Y¹ eine direkte Bindung ist oder -(CR⁹R¹⁰)ₛY²- darstellt, wobei s eine ganze Zahl von 0 bis 3 ist, R⁹ und R¹⁰ dieselben wie oben definiert sind und Y² aus der Gruppe, bestehend aus -O-, -C(O)- und -C(O)O-, ausgewählt ist, und
R¹² aus der Gruppe, bestehend aus Wasserstoff, Halogen, C₁-C₆-Alkyl und -(CH₂)ₜB¹-R¹³, ausgewählt ist, wobei t eine ganze Zahl von 0 bis 3 ist, B¹ einen 5-6-gliedrigen Heterozyklus darstellt, der ein oder zwei Heteroatome, ausgewählt aus N-, O- und S-Atomen, enthält, oder C₆-C₁₀-Aryl darstellt, R¹³ Wasserstoff, Cyano, Halogen, Hydroxy, Oxo, Thiol, Carboxy oder Carboxy-C₁-C₆-alkyl darstellt, mit der Maßgabe, dass R⁴ Wasserstoff oder C₁-C₆-Alkyl ist, wenn X N ist,
wobei Alkyl, Alkoxy, Aryl, Cycloalkyl, Heterozyklus und Heteroaryl optional substituiert sein können und die Substituenten eines oder mehrere, ausgewählt aus der Gruppe, bestehend aus Hydroxy, C₁-C₆-Alkylamino, Di(C₁-C₆-alkyl)amino, Carboxy, C₁-C₆-Alkyl, C₁-C₆-Alkoxy, Carboxy-C₁-C₆-alkyl und Oxo, sind, und 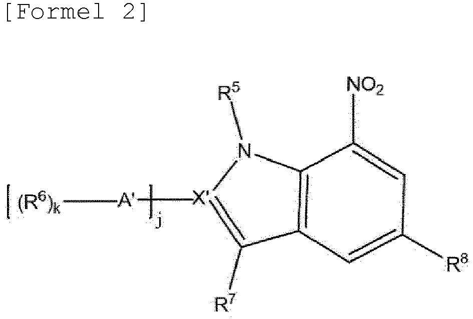
wobei in der obigen Formel 2
k eine ganze Zahl von 1 bis 3 ist,
j 0 oder 1 ist,
A' C₃-C₈-Cycloalkyl, Phenyl oder 5-6-gliedriges/gliedriger Heteroaryl oder Heterozyklus darstellt, das/der jeweils 1 oder 2 Heteroatome, ausgewählt unter N-, O- und S-Atomen, enthält,
X' C oder N darstellt, mit der Maßgabe, dass j 0 ist, wenn X' N ist, und j 1 ist, wenn X' C ist,
R⁵ Wasserstoff, C₁-C₆-Alkyl oder -(CH₂)ₑNR¹⁴R¹⁵ darstellt, wobei e eine ganze Zahl von 2 bis 5 ist, R¹⁴ und R¹⁵ jeweils unabhängig Wasserstoff oder C₁-C₃-Alkyl darstellen, mit der Maßgabe, dass R⁵ Wasserstoff ist, wenn X' N ist,
R⁶ Wasserstoff, Halogen oder C₁-C₆-Alkoxy darstellt oder -(CH₂)_{f}C(O)₂R¹⁴, -(CH₂)_{f}OR¹⁴, -(CH₂)_{f}NR¹⁴R¹⁵, -NHR¹⁶, -N(H)S(O)₂R¹⁴ oder -NHC(O)₂R¹⁶ darstellt oder -(CH₂)_{f}-Heterozyklus-R¹⁶ darstellt, worin der Heterozyklus ein 5-6-gliedriger Ring ist, der ein oder zwei Heteroatome, ausgewählt unter N-, O- und S-Atomen, enthält, wobei f eine ganze Zahl von 0 bis 3 ist, R¹⁴ und R¹⁵ dieselben wie oben definiert sind und R¹⁶ Wasserstoff, Oxo, C₁-C₆-Alkylcarbonyl, C₁-C₆-Alkoxy oder C₁-C₆-Alkyl oder einen 5-6-gliedrigen Heterozyklus darstellt, der ein oder zwei Stickstoffatome als ein Heteroatom enthält,
R⁷ Wasserstoff, Halogen, C₁-C₆-Alkyl oder Phenyl oder -(CH₂)_{g}-Heterozyklus darstellt, wobei der Heterozyklus ein 5-6-gliedriger Ring ist, der ein oder zwei Heteroatome, ausgewählt aus N- und O-Atomen enthält, wobei g eine ganze Zahl von 1 bis 3 ist, mit der Maßgabe, dass R⁷ Wasserstoff oder Phenyl ist, wenn X' N ist,
R⁸ -Y³R¹⁷ darstellt, wobei Y³ eine direkte Bindung ist oder -(CR¹⁴R¹⁵)ₕY⁴- darstellt, wobei h eine ganze Zahl von 0 bis 3 ist, R¹⁴ und R¹⁵ dieselben wie oben definiert sind und Y⁴ aus der Gruppe, bestehend aus -O-, -C(O)- und -C(O)O-, ausgewählt ist, und
R¹⁷ aus der Gruppe, bestehend aus Wasserstoff, Halogen, C₁-C₆-Alkyl und -(CH₂)ᵢB²-R¹⁸, ausgewählt ist, wobei i eine ganze Zahl von 0 bis 3 ist, B² einen 5-6-gliedrigen Heterozyklus darstellt, der ein oder zwei Heteroatome, ausgewählt unter N-, 0- und S-Atomen, enthält, oder C₆-C₁₀-Alkyl darstellt, R¹⁸ Wasserstoff, Cyano, Halogen, Hydroxy, Oxo, Thiol, Carboxy oder Carboxy-C₁-C₆-alkyl darstellt, mit der Maßgabe, dass R⁸ Wasserstoff oder C₁-C₆-Alkyl ist, wenn X' N ist,
wobei Alkyl, Alkoxy, Aryl, Cycloalkyl, Heterozyklus und Heteroaryl optional substituiert sein können und die Substituenten eines oder mehrere, ausgewählt aus der Gruppe, bestehend aus Hydroxy, C₁-C₆-Alkylamino, Di(C₁-C₆-alkyl)amino, Carboxy, C₁-C₆-Alkyl, C₁-C₆-Alkoxy, Carboxy-C₁-C₆-alkyl und Oxo sind.

2. Verfahren nach Anspruch 1, wobei das Eisen(III)-chlorid-Hydrat Eisen(III)-chlorid-Hexahydrat (FeCl₂·6H₂O) ist.

3. Verfahren nach Anspruch 1, wobei der Reduktionsschritt bei einer Temperatur von 0°C bis 65°C durchgeführt wird.

4. Verfahren nach Anspruch 1, wobei die durch die obige Formel 1 dargestellte Verbindung durch die nachstehende Formel (1a) oder (1b) dargestellt ist: 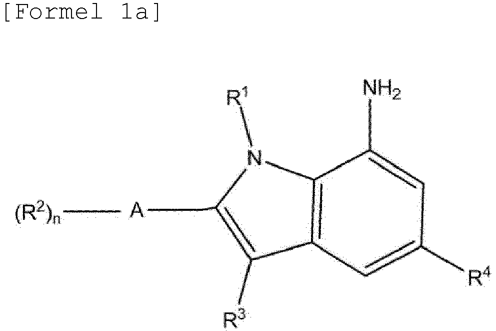 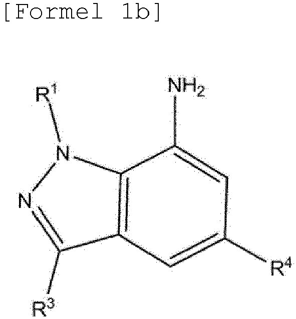 wobei in den obigen Formeln n, A, R¹, R², R³ und R⁴ dieselben wie in Anspruch 1 definiert sind.

5. Verfahren nach Anspruch 1, wobei R¹ Wasserstoff, C₁-C₆-Alkyl oder Di(C₁-C₃-alkyl)amino-C₂-C₃-alkyl darstellt.

6. Verfahren nach Anspruch 1, wobei Wasserstoff, Halogen, Carboxy-C₁-C₃-alkyl, C₁-C₃-Alkoxycarbonyl, C₁-C₃-Alkoxycarbonyl-C₁-C₃-alkyl, Hydroxy-C₁-C₃-alkyl gegebenenfalls substituiert durch eine Oxogruppe, C₁-C₃-Alkoxy, -(CH₂)ₚNR⁹R¹⁰, -NHR¹¹, -N(H)S(O)₂R⁹ oder -NHC(O)₂R¹¹, oder -(CH₂)ₚ-Heterozyklus-R¹¹ sein kann, wobei Heterozyklus, p, R⁹, R¹⁰ und R¹¹ dieselben wie in Anspruch 1 definiert sind.

7. Verfahren nach Anspruch 1, wobei R³ Wasserstoff, Methyl oder Halogen, Phenyl, optional substituiert durch C₁-C₃-Alkoxy oder Heterocyclyl-C₁-C₃-alkylen darstellt, worin der Heterozyklus ein 5-6-gliedriger Ring ist, der ein oder zwei Heteroatome, ausgewählt aus N- und O-Atomen, enthält und optional mit einer oder zwei Oxogruppen substituiert ist.

8. Verfahren nach Anspruch 1, wobei Y¹ aus der Gruppe, bestehend aus einer direkten Bindung, -O-, -C(O)- und -CH₂C(O)-, ausgewählt ist.

9. Verfahren nach Anspruch 1, wobei R¹² aus der Gruppe, bestehend aus Wasserstoff, Methyl, Ethyl, Phenyl, Fluor, Chlor, 2-Carboxypyrrolidin-1-yl, Pyrrolidin-1-yl, 4-Essigsäure-1,3-thiazolin-2-yl, -CH₂-(1,1-Dioxothiomorpholin-4-yl) und -CH₂-(2-Oxopiperazin-4-yl), ausgewählt ist.

10. Verfahren nach Anspruch 1, wobei die durch Formel 1 dargestellte Verbindung (4-((7-Amino-2-phenyl-1H-indol-5-yl)methyl)thiomorpholin-1,1-dioxid ist und die durch Formel 2 dargestellte Verbindung (4-((7-Nitro-2-phenyl-1H-indol-5-yl)methyl)thiomorpholin-1,1-dioxid ist.

11. Verfahren zur Herstellung einer durch die nachstehende Formel 3 dargestellten Verbindung, wobei das Verfahren das Verfahren nach Anspruch 1 umfasst, gefolgt von einem Schritt zum Reagieren der durch Formel 1 dargestellten Verbindung mit einem Keton oder einem Aldehyd: 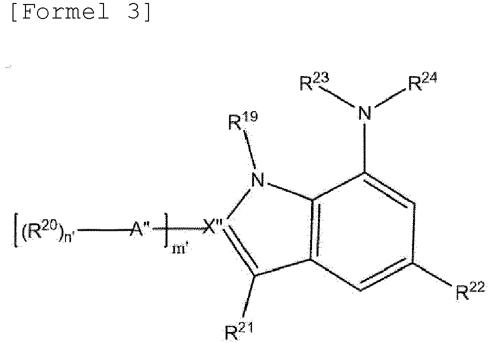
wobei in der obigen Formel 3
n' eine ganze Zahl von 1 bis 3 ist,
m' 0 oder 1 ist,
A" C₃-C₈-Cycloalkyl, Phenyl oder 5-6-gliedriges/gliedriger Heteroaryl oder Heterozyklus darstellt, das/der jeweils ein oder zwei Heteroatome, ausgewählt unter N-, O- und S-Atomen, enthält,
X" C oder N darstellt, mit der Maßgabe, dass m' 0 ist, wenn X" N ist, und m' 1 ist, wenn X" C ist,
R¹⁹ Wasserstoff, C₁-C₆-Alkyl oder -(CH₂)ᵤNR²⁵R²⁶ darstellt, wobei u eine ganze Zahl von 2 bis 5 ist, R²⁵ und R²⁶ jeweils unabhängig Wasserstoff oder C₁-C₃-Alkyl darstellen, mit der Maßgabe, dass R¹⁹ Wasserstoff ist, wenn X" N ist,
R²⁰ Wasserstoff, Halogen oder C₁-C₆-Alkoxy darstellt oder -(CH₂)ᵥC(O)₂R²⁵, -(CH₂)ᵥOR²⁵, -(CH₂)ᵥNR²⁵R²⁶, -NHR²⁷, -N(H)S(O)₂R²⁵ oder -NHC(O)₂R²⁷ darstellt oder -(CH₂)ᵥ-Heterozyklus-R²⁷ darstellt, worin der Heterozyklus ein 5-6-gliedriger Ring ist, der ein oder zwei Heteroatome, ausgewählt unter N-, O- und S-Atomen, enthält, wobei v eine ganze Zahl von 0 bis 3 ist, R²⁵ und R²⁶ dieselben wie oben definiert sind und R²⁷ Wasserstoff, Oxo, C₁-C₆-Alkylcarbonyl, C₁-C₆-Alkoxy oder C₁-C₆-Alkyl darstellt, oder einen 5-6-gliedrigen Heterozyklus, der ein oder zwei Stickstoffatome als ein Heteroatom enthält,
R²¹ Wasserstoff, Halogen, C₁-C₆-Alkyl oder Phenyl oder -(CH₂)_{w}-Heterozyklus, worin der Heterozyklus ein 5-6-gliedriger Ring ist, der ein oder zwei Heteroatome, ausgewählt unter N- und O-Atomen, enthält, wobei w eine ganze Zahl von 1 bis 3 ist, mit der Maßgabe, dass R²¹ Wasserstoff oder Phenyl ist, wenn X" N ist,
R²² Y⁵R²⁸ darstellt, worin Y⁵ eine direkte Bindung oder -(CR²⁵R²⁶)ₓY⁶- ist, worin x eine ganze Zahl von 0 bis 3 ist, R²⁵ und R²⁶ dieselben wie oben definiert sind und Y⁶ aus der Gruppe, bestehend aus -O-, -C(O)- und -C(O)O-, ausgewählt ist,
R²⁸ aus der Gruppe, bestehend aus Wasserstoff, Halogen, C₁-C₆-Alkyl und -(CH₂)_{y}B³-R²⁹, ausgewählt ist, wobei y eine ganze Zahl von 0 bis 3 ist, B³ einen 5-6-gliedrigen Heterozyklus darstellt, der ein oder zwei Heteroatome, ausgewählt unter N-, O- und S-Atomen, enthält, oder C₆-C₁₀-Aryl darstellt, R²⁹ Wasserstoff, Cyano, Halogen, Hydroxy, Oxo, Thiol, Carboxy oder Carboxy-C₁-C₆-alkyl dargestellt, mit der Maßgabe, dass R²² Wasserstoff oder C₁-C₆-Alkyl ist, wenn X" N ist,
R²³ Wasserstoff, C₁-C₆-Alkyl, C₃-C₆-Cycloalkyl, Heterozyklus oder Heterocyclyl-C₁-C₆-alkyl darstellt, wobei der Heterozyklus ein 3-8-gliedriger Ring ist, der ein bis drei Heteroatome, ausgewählt unter N- und O-Atomen, enthält, mit der Maßgabe, dass R²³ Wasserstoff ist, wenn X" N ist, und
R²⁴ -(CR²⁵R²⁶)ₒ-Z-D-W-R³⁰ darstellt, wobei Z eine direkte Bindung ist oder aus der Gruppe, bestehend aus -C(O)- und -C(O)O-, ausgewählt ist, D eine direkte Bindung, C₄-C₆-Cycloalkyl, 5-6-gliedriges Heteroaryl, enthaltend ein oder zwei N-Atome, oder einen 5-6-gliedrigen Heterozyklus darstellt, der ein oder zwei Heteroatome, ausgewählt und N-, O- und S-Atomen, enthält, W eine direkte Bindung oder -NR²⁵-, -C(0)-, -C(O)O-, -C(O)NR³¹- oder -S(O)_{z}- darstellt, R³¹ Wasserstoff, C₁-C₃-Alkyl oder C₆-C₁₀-Aryl darstellt, z eine ganze Zahl von 1 oder 2 ist, o eine ganze Zahl von 0 bis 3 ist, R²⁵ und R²⁶ dieselben wie oben definiert sind, R³⁰ Wasserstoff, Hydroxy, C₁-C₆-Alkyl, einen 5-6-gliedrigen Heterozyklus, der ein bis drei Heteroatome, ausgewählt unter N-, O- und S-Atomen, enthält, oder C₆-C₁₀-Ar-C₁-C₆-Alkyl darstellt, mit der Maßgabe, dass, wenn X" N ist, R²⁴ C₄-C₆-Cycloalkyl oder einen 5-6-gliedrigen Heterozyklus darstellt, der ein oder zwei Heteroatome, ausgewählt unter N-, O- und S-Atomen, enthält,
wobei Alkyl, Alkoxy, Aryl, Cycloalkyl, Heterozyklus und Heteroaryl optional substituiert sein können und die Substituenten eines oder mehrere, ausgewählt aus der Gruppe, bestehend aus Hydroxy, C₁-C₆-Alkylamino, Di(C₁-C₆-alkyl)amino, Carboxy, C₁-C₆-Alkyl, C₁-C₆-Alkoxy, Carboxy-C₁-C₆-alkyl und Oxo, sind.

## Revendications

1. Procédé de préparation d'un composé représenté par la formule 1 ci-dessous, le procédé comprenant une étape de réduction d'un composé représenté par la formule 2 ci-dessous en présence de chlorure de fer(lll) hydrate,
dans lequel l'étape de réduction est mise en oeuvre en comprenant un agent réducteur supplémentaire, et
dans lequel l'agent réducteur est au moins un élément sélectionné dans le groupe constitué de NaBH₄, 9-borabicyclo[3.3.1]nonane (9-BBN), BH₃SMe₂ et LiBH₄ : 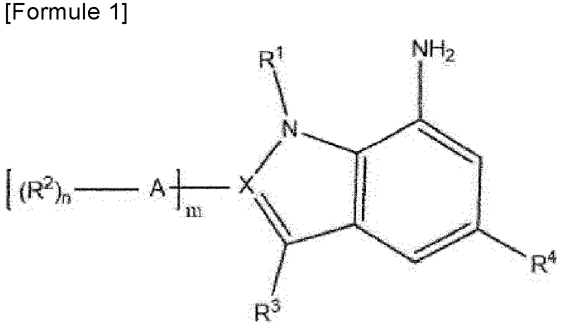
dans lequel, dans la formule 1 ci-dessus,
n est un nombre entier de 1 à 3,
m est 0 ou 1,
A représente un cycloalkyle en C₃-C₈, un phényle ou un hétéroaryle ou hétérocycle à 5-6 chaînons qui contiennent chacun un ou deux hétéroatomes sélectionnés parmi des atomes de N, O et S,
X représente C ou N, à condition que m soit 0 lorsque X est N et que m soit 1 lorsque X est C,
R¹ représente un hydrogène, un alkyle en C₁-C₆ ou -(CH₂)ᵣNR⁹R¹⁰, dans lequel r est un nombre entier de 2 à 5, R⁹ et R¹⁰ représentent chacun indépendamment un hydrogène ou un alkyle en C₁-C₃, à condition que R¹ soit un hydrogène lorsque X est N,
R² représente un hydrogène, un halogène ou un alcoxy en C₁-C₆, ou représente -(CH₂)ₚC(O)₂R⁹, -(CH₂)ₚOR⁹, -(CH₂)ₚNR⁹R¹⁰, -NHR¹¹,-N(H)S(O)₂R⁹ ou -NHC(O)₂R¹¹, ou représente un -(CH₂)ₚ-hétérocycle-R¹¹ dans lequel l'hétérocycle est un cycle à 5-6 chaînons contenant un ou deux hétéroatomes sélectionnés parmi des atomes de N, O et S, dans lequel p est un nombre entier de 0 à 3, R⁹ et R¹⁰ sont tels que définis ci-dessus et R¹¹ représente un hydrogène, un oxo, un alkylcarbonyle en C₁-C₆, un alcoxy en C₁-C₆ ou un alkyle en C₁-C₆, ou un hétérocycle à 5-6 chaînons contenant un ou deux atomes d'azote en tant qu'hétéroatome,
R³ représente un hydrogène, un halogène, un alkyle en C₁-C₆ ou un phényle, ou un -(CH₂)_{q}-hétérocycle, dans lequel l'hétérocycle étant un cycle à 5-6 chaînons contenant un ou deux hétéroatomes sélectionnés parmi des atomes de N et O, dans lequel q est un nombre entier de 1 à 3, à condition que R³ soit un hydrogène ou un phényle lorsque X est N,
R⁴ représente -Y¹ R¹², dans lequel Y¹ est une liaison directe ou représente -(CR⁹R¹⁰) sY²-, dans lequel s est un nombre entier de 0 à 3, R⁹ et R¹⁰ sont tels que définis ci-dessus, et Y² est sélectionné dans le groupe constitué de -O-, -C(O)-et -C(O)O-, et
R¹² est sélectionné dans le groupe constitué d'un hydrogène, d'un halogène, d'un alkyle en C₁-C₆ et de -(CH₂)ₜB¹-R¹³, dans lequel t est un nombre entier de 0 à 3, B¹ représente un hétérocycle à 5-6 chaînons contenant un ou deux hétéroatomes sélectionnés parmi des atomes de N, O et S, ou représente un aryle en C₆-C₁₀, R¹³ représente un hydrogène, un cyano, un halogène, un hydroxy, un oxo, un thiol, un carboxy ou un carboxy-C₁-C₆ alkyle, à condition que R⁴ soit un hydrogène ou un C₁-C₆ alkyle lorsque X est N,
dans lequel un alkyle, un alcoxy, un aryle, un cycloalkyle, un hétérocycle et un hétéroaryle peuvent être facultativement substitués, et les substituants sont un ou plusieurs éléments sélectionnés dans le groupe constitué d'un hydroxy, d'un C₁-C₆ alkylamino, d'un di(C₁-C₆ alkyle)amino, d'un carboxy, d'un alkyle en C₁-C₆, d'un alcoxy en C₁-C₆, d'un carboxy-C₁-C₆ alkyle et d'un oxo, et 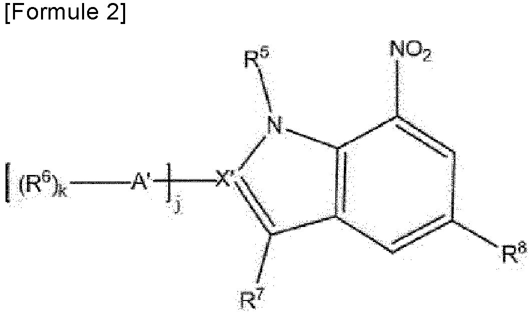
dans lequel, dans la formule 2 ci-dessus,
k est un nombre entier de 1 à 3,
j est 0 ou 1,
A' représente un C₃-C₈ cycloalkyle, un phényle ou un hétéroaryle ou hétérocycle à 5-6 chaînons qui contiennent chacun 1 ou 2 hétéroatomes sélectionnés parmi des atomes de N, O et S,
X' représente C ou N, à condition que j soit 0 lorsque X' est N et que j soit 1 lorsque X' est C,
R⁵ représente un hydrogène, un alkyle en C₁-C₆ ou -(CH₂)ₑNR¹⁴R¹⁵, dans lequel e est un nombre entier de 2 à 5, R¹⁴ et R¹⁵ représentent chacun indépendamment un hydrogène ou un alkyle en C₁-C₃, à condition que R⁵ soit un hydrogène lorsque X' est N,
R⁶ représente un hydrogène, un halogène ou un C₁-C₆ alcoxy, ou représente -(CH₂)_{f}C(O)₂R¹⁴, -(CH₂)_{f}OR¹⁴, -(CH₂)rNR¹⁴R¹⁵, -NHR¹⁶, -N(H)S(O)₂R¹⁴ ou -NHC(O)₂R¹⁶, ou représente un -(CH₂)_{f}-hétérocycle-R¹⁶ dans lequel l'hétérocycle est un cycle à 5-6 chaînons contenant un ou deux hétéroatomes sélectionnés parmi des atomes de N, O et S, dans lequel f est un nombre entier de 0 à 3, R¹⁴ et R¹⁵sont tels que définis ci-dessus et R¹⁶ représente un hydrogène, un oxo, un alkylcarbonyle en C₁-C₆, un alcoxy en C₁-C₆ ou un alkyle en C₁-C₆, ou un hétérocycle à 5-6 chaînons contenant un ou deux atomes d'azote en tant qu'hétéroatome,
R⁷ représente un hydrogène, un halogène, un alkyle en C₁-C₆ ou un phényle, ou un -(CH₂)_{g}-hétérocycle dans lequel l'hétérocycle est un cycle à 5-6 chaînons contenant un ou deux hétéroatomes sélectionnés parmi des atomes de N et O, dans lequel g est un nombre entier de 1 à 3, à condition que R⁷ soit un hydrogène ou un phényle lorsque X' est N,
R⁸ représente -Y³R¹⁷, dans lequel Y³ est une liaison directe ou représente -(CR¹⁴R¹⁵)ₕY⁴-, dans lequel h est un nombre entier de 0 à 3, R¹⁴ et R¹⁵ sont tels que définis ci-dessus, et Y⁴ est sélectionné dans le groupe constitué de -O-, -C(O)-et -C(O)O-, et
R¹⁷ est sélectionné dans le groupe constitué d'un hydrogène, d'un halogène, d'un alkyle en C₁-C₆ et de -(CH₂)ᵢB²-R¹⁸, dans lequel i est un nombre entier de 0 à 3, B² représente un hétérocycle à 5-6 chaînons contenant un ou deux hétéroatomes sélectionnés parmi des atomes de N, O et S, ou représente un aryle en C₆-C₁₀, R¹⁸ représente un hydrogène, un cyano, un halogène, un hydroxy, un oxo, un thiol, un carboxy ou un carboxy-C₁-C₆ alkyle, à condition que R⁸ soit un hydrogène ou un alkyle en C₁-C₆ lorsque X' est N,
dans lequel un alkyle, un alcoxy, un aryle, un cycloalkyle, un hétérocycle et un hétéroaryle peuvent être facultativement substitués, et les substituants sont un ou plusieurs éléments sélectionnés dans le groupe constitué d'un hydroxy, d'un alkylamino en C₁-C₆, d'un di(C₁-C₆ alkyle)amino, d'un carboxy, d'un alkyle en C₁-C₆, d'un alcoxy en C₁-C₆, d'un carboxy-C₁-C₆ alkyle et d'un oxo.

2. Procédé selon la revendication 1, dans lequel le chlorure de fer(Ill) hydrate est un chlorure ferrique hexahydraté (FeCl₃·6H₂O).

3. Procédé selon la revendication 1, dans lequel l'étape de réduction est réalisée à une température de 0 °C à 65 °C.

4. Procédé selon la revendication 1, dans lequel le composé représenté par la formule 1 ci-dessus est représenté par la formule (1a) ou (1b) ci-dessous : 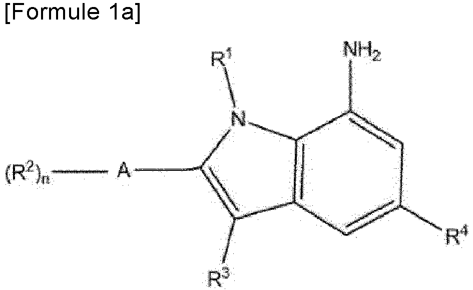 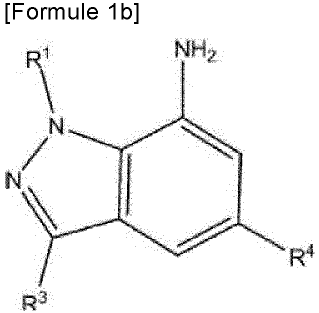 dans lequel, dans les formules ci-dessus, n, A, R¹, R², R³ et R⁴ sont tels que définis dans la revendication 1.

5. Procédé selon la revendication 1, dans lequel R¹ représente un hydrogène, un C₁-C₆ alkyle ou un di(C₁-C₃ alkyle)amino-C₂-C₃ alkyle.

6. Procédé selon la revendication 1, dans lequel R² représente un hydrogène, un halogène, un carboxy, un carboxy-C₁-C₃ alkyle, un C₁-C₃ alcoxycarbonyle, un C₁-C₃ alcoxycarbonyle-C₁-C₃ alkyle, un hydroxy-C₁-C₃ alkyle facultativement substitué par un groupe oxo, un C₁-C₃ alcoxy, (CH₂)ₚNR⁹R¹⁰, -NHR¹¹, -N(H)S(O)₂R⁹ ou -NHC(O)₂R¹¹ ou peut être un -(CH₂)ₚ-hétérocycle-R¹¹, dans lequel l'hétérocycle, p, R⁹, R¹⁰ et R¹¹ sont tels que définis dans la revendication 1.

7. Procédé selon la revendication 1, dans lequel R³ représente un hydrogène, un méthyle ou un halogène, un phényle facultativement substitué par un C₁-C₃ alcoxy ou un hétérocyclyle-C₁-C₃ alkylène dans lequel l'hétérocycle est un cycle à 5-6 chaînons contenant un ou deux hétéroatomes sélectionnés parmi des atomes de N et O et facultativement substitués par un ou deux groupes oxo.

8. Procédé selon la revendication 1, dans lequel Y¹ est sélectionné dans le groupe constitué d'une liaison directe, de -O-, de -C(O)- et de -CH₂C(O)-.

9. Procédé selon la revendication 1, dans lequel R¹² est sélectionné dans le groupe constitué d'un hydrogène, d'un méthyle, d'un éthyle, d'un phényle, d'un fluoro, d'un chloro, d'un 2-carboxy-pyrrolidin-1-yle, d'un pyrrolidin-1-yle, d'un acide 4-acétique-1,3-thiazolin-2-yle, d'un -CH₂-(1,1-dioxo-thiomorpholin-4-yle) et d'un -CH₂-(2-oxopipérazin-4-yle).

10. Procédé selon la revendication 1, dans lequel le composé représenté par la formule 1 est du 1,1-dioxyde de (4-((7-amino-2-phényl-1H-indol-5-yl)méthyl)thiomorpholine, et le composé représenté par la formule 2 est du 1,1-dioxyde de (4-((7-nitro-2-phényl-1H-indol-5-yl)méthyl)thiomorpholine.

11. Procédé de préparation d'un composé représenté par la formule 3 ci-dessous, le procédé comprenant le procédé selon la revendication 1 suivi d'une étape de réaction du composé représenté par la formule 1 avec une cétone ou un aldéhyde : 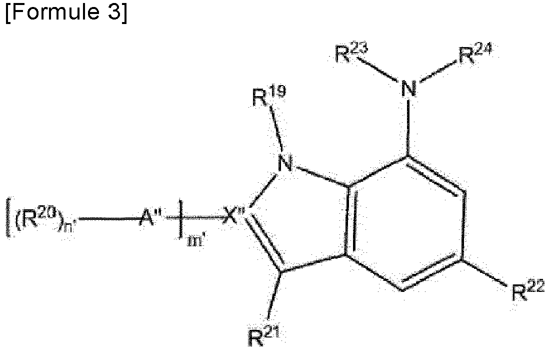
dans lequel, dans la formule 3 ci-dessus,
n' est un nombre entier de 1 à 3,
m' est 0 ou 1,
A" représente un C₃-C₈ cycloalkyle, un phényle ou un hétéroaryle ou hétérocycle à 5-6 chaînons qui contiennent chacun un ou deux hétéroatomes sélectionnés parmi des atomes de N, O et S,
X" représente C ou N, à condition que m' soit 0 lorsque X" est N et que m' soit 1 lorsque X" est C,
R¹⁹ représente un hydrogène, un alkyle en C₁-C₆ ou -(CH₂)_{U}NR²⁵R²⁶, dans lequel u est un nombre entier de 2 à 5, R²⁵ et R²⁶ représentent chacun indépendamment un hydrogène ou un alkyle en C₁-C₃, à condition que R¹⁹ soit un hydrogène lorsque X" est N,
R²⁰ représente un hydrogène, un halogène ou un alcoxy en C₁-C₆, ou représente -(CH₂)ᵥC(O)₂R²⁵, -(CH₂)ᵥOR²⁵, -(CH₂)ᵥNR²⁵R²⁶, -NHR²⁷, -N(H)S(O)₂R²⁵ ou -NHC(O)₂R²⁷, ou représente un (CH₂)ᵥ-hétérocycle-R²⁷ dans lequel l'hétérocycle est un cycle à 5-6 chaînons contenant un ou deux hétéroatomes sélectionnés parmi des atomes de N, O et S, dans lequel v est un nombre entier de 0 à 3, R²⁵ et R²⁶ sont tels que définis ci-dessus et R²⁷ représente un hydrogène, un oxo, un alkylcarbonyle en C₁-C₆, un alcoxy en C₁-C₆ ou un alkyle en C₁-C₆, ou un hétérocycle à 5-6 chaînons contenant un ou deux atomes d'azote en tant qu'hétéroatome,
R²¹ représente un hydrogène, un halogène, un alkyle en C₁-C₆ ou un phényle, ou un -(CH₂)_{w}-hétérocycle dans lequel l'hétérocycle est un cycle à 5-6 chaînons contenant un ou deux hétéroatomes sélectionnés parmi des atomes de N et O, dans lequel w est un nombre entier de 1 à 3, à condition que R²¹ soit un hydrogène ou un phényle lorsque X" est N,
R²² représente -Y⁵R²⁸, dans lequel Y⁵ est une liaison directe ou représente -(CR²⁵R²⁶)ₓY⁶-, dans lequel x est un nombre entier de 0 à 3, R²⁵ et R²⁶ sont tels que définis ci-dessus, et Y⁶ est sélectionné dans le groupe constitué de -O-, -C(O)-et -C(O)O-,
R²⁸ est sélectionné dans le groupe constitué d'un hydrogène, d'un halogène, d'un C₁-C₆ alkyle et de -(CH₂)_{y}B³-R²⁹, dans lequel y est un nombre entier de 0 à 3, B³ représente un hétérocycle à 5-6 chaînons contenant un ou deux hétéroatomes sélectionnés parmi des atomes de N, O et S, ou représente un aryle en C₆-C₁₀, R²⁹ représente un hydrogène, un cyano, un halogène, un hydroxy, un oxo, un thiol, un carboxy ou un carboxy-C₁-C₆ alkyle, à condition que R²² soit un hydrogène ou un alkyle en C₁-C₆ lorsque X" est N,
R²³ représente un hydrogène, un alkyle en C₁-C₆ ou un cycloalkyle en C₃-C₆, un hétérocycle ou un hétérocyclyle-C₁-C₆ alkyle, dans lequel l'hétérocycle est un cycle à 3-8 chaînons contenant un à trois hétéroatomes sélectionnés parmi des atomes de N et O, à condition que R²³ soit un hydrogène lorsque X" est N, et
R²⁴ représente -(CR²⁵R²⁶)ₒ-Z-D-W-R³⁰, dans lequel Z représente une liaison directe ou est sélectionné dans le groupe constitué de -C(O)- et -C(0)0-, D représente une liaison directe, un C₄-C₆ cycloalkyle, un hétéroaryle à 5-6 chaînons contenant un ou deux atomes de N, ou un hétérocycle à 5-6 chaînons contenant un ou deux hétéroatomes sélectionnés parmi des atomes de N, O et S, W représente une liaison directe, ou -NR²⁵-, -C(O)-, -C(O)O-, -C(O)NR³¹- ou -S(O)_{z}-, R³¹ représente un hydrogène, un alkyle en C₁-C₃ ou un aryle en C₆-C₁₀, z est un nombre entier valant 1 ou 2, o est un nombre entier de 0 à 3, R²⁵ et R²⁶ sont tels que définis ci-dessus, R³⁰ représente un hydrogène, un hydroxy, un alkyle en C₁-C₆, un hétérocycle à 5-6 chaînons contenant trois hétéroatomes sélectionnés parmi des atomes de N, O et S, ou un C₆-C₁₀ Ar-C₁-C₆ alkyle, à condition que, lorsque X" est N, R²⁴ représente un C₄-C₆ cycloalkyle ou un hétérocycle à 5-6 chaînons contenant un ou deux hétéroatomes sélectionnés parmi des atomes de N, O et S,
dans lequel un alkyle, un alcoxy, un aryle, un cycloalkyle, un hétérocycle et un hétéroaryle peuvent être facultativement substitués, et les substituants sont un ou plusieurs éléments sélectionnés dans le groupe constitué d'un hydroxy, d'un alkylamino C₁-C₆, d'un di(C₁-C₆ alkyle)amino, d'un carboxy, d'un alkyle en C₁-C₆, d'un alcoxy en C₁-C₆, d'un carboxy-C₁-C₆ alkyle et d'un oxo.
